(19) Europäisches Patentamt
European Patent Office
Office européen des brevets

(11) EP 0 514 267 B1

(12) FASCICULE DE BREVET EUROPEEN

(45) Date de publication et mention de la délivrance du brevet: **26.06.1996 Bulletin 1996/26**

(51) Int Cl.$^{6}$: **C07D 498/04**, A61K 31/535 // (C07D498/04, 265:00, 221:00)

(21) Numéro de dépôt: **92401312.1**

(22) Date de dépôt: **14.05.1992**

(54) **Nouveaux dérivés amidiques de 1-amino octahydropyrido (2,1-c) (1,4) oxazine, leurs procédés de préparation et les compositions pharmaceutiques qui les contiennent**

Amidderivate von 1-Amino-Octahydropyrido[2,1-c][1,4]oxazin, Verfahren zu ihrer Herstellung und sie enthaltende pharmazeutische Zusammensetzungen

Amide derivatives of 1-amino-octahydropyrido[2,1-c][1,4]oxazine, processes for their preparation and pharmaceutical compositions containing them

(84) Etats contractants désignés:
**AT BE CH DE DK ES FR GB GR IT LI LU MC NL PT SE**

(30) Priorité: **14.05.1991 FR 9105765**

(43) Date de publication de la demande:
**19.11.1992 Bulletin 1992/47**

(73) Titulaire: **ADIR ET COMPAGNIE**
**F-92415 Courbevoie Cédex (FR)**

(72) Inventeurs:
- **Husson, Henri Philippe**
**F-78460 Chevreuse (FR)**
- **Quirion, Jean Charles**
**F-91190 Gif Sur Yvette (FR)**
- **Bonin, Martine**
**F-91190 Gif Sur Yvette (FR)**
- **Guardiola, Béatrice**
**F-92200 Neuilly Sur Seine (FR)**
- **Adam, Gérard**
**F-78600 Le Mesnil Le Roi (FR)**
- **Renard, Pierre**
**F-78000 Versailles (FR)**

(56) Documents cités:
**EP-A- 0 034 015**
**EP-A- 0 057 536**
**GB-A- 1 593 146**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 514 267 B1

## Description

La présente invention concerne de nouveaux dérivés amidiques de 1-amino octahydropyrido [2,1-c] [1,4] oxazines, leur procédé de préparation et les compositions pharmaceutiques qui les contiennent.

Des dérivés benzamidiques obtenus à partir de 9-amino octahydropyrido [2,1-c] [1,4] oxazines (brevet EP 57536 A1 11/08/82) et de 8-amino octahydropyrido [2,1-c] [1,4] oxazines (brevet EP 34015 A2 19/08/81) sont déjà connus pour leur capacité à améliorer la motilité du tractus gastro intestinal.

Les dérivés de la demanderesse possèdent une structure de 1-amino octahydropyrido [2,1-c] [1,4] oxazine entièrement nouvelle, pouvant être obtenue de manière stéréospécifique, et sont pour certains d'entre eux, dotés de remarquables propriétés psychotropes en étant fortement antidépresseurs, antipsychotiques, neuroleptiques.

Plus spécifiquement l'invention concerne les 1-acylamino octahydropyrido [2,1-c] [1,4] oxazines de formule générale **(I)** :

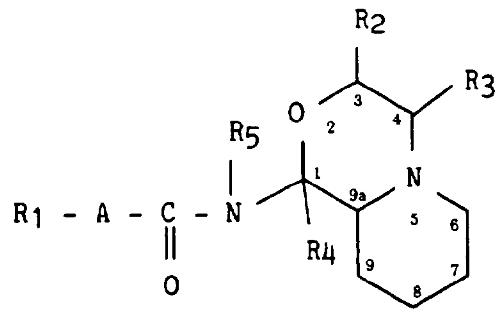

(I)

dans laquelle :

- $R_1$ représente un hydrogène ou un système mono ou bicyclique éventuellement substitué choisi parmi phényl, naphtyl, pyridyl, thiényl, furyl, quinolyl, isoquinolyl, indolyl, indolinyl, perhydroindolyl, benzofuryl,
- A représente une liaison $\alpha$ ou un alkyl de 1 à 4 atomes de carbone, linéaire ou ramifié, saturé ou insaturé, avec la réserve que lorsque $R_1$ représente un hydrogène, A ne peut représenter une liaison $\sigma$,
- $R_2$ et $R_3$ représentent chacun indépendamment l'un de l'autre
  - un hydrogène,
  - un alkyl de 1 à 6 atomes de carbone linéaire ou ramifié et éventuellement substitué,
  - un groupement - $(CH2)_nB$ avec n pouvant prendre les valeurs 0,1,2,3 et B représentant un système monocyclique substitué ou non de 5 à 7 sommets, saturé, insaturé ou aromatique,

  ou $R_2$ et $R_3$ forment ensemble avec les atomes de carbone auquels ils sont liés un système cyclique saturé éventuellement substitué de 5 à 7 sommets,
- $R_4$ et $R_5$ représentent chacun indépendamment l'un de l'autre un hydrogène, un alkyl de 1 à 4 atomes de carbone linéaire ou ramifié ou un groupement - $(CH_2)_nB$ tel que défini dans la description de $R_2$ et $R_3$,

le terme substitué associé aux systèmes "mono ou bicyclique" signifie que ces cycles peuvent être substitués par un ou des groupements (maximum 4 ), identiques ou différents, alkyle inférieur de 1 à 4 atomes de carbone ramifié ou non, nitro, amino, alkoxy inférieur de 1 à 4 atomes de carbone ramifié ou non, hydroxy, trifluorométhyle, halogène ou $SO_2R_6$ ,
le terme substitué associé à l'expression alkyle signifie que ce groupement peut être substitué par un ou des groupements alkoxy inférieur de 1 à 4 atomes de carbone linéaire ou ramifié,

- $R_6$ représente un alkyle inférieur de 1 à 4 atomes de carbone ramifié ou non, un phényl (éventuellement substitué par un ou plusieurs groupements alkyl ou alkoxy inférieur de 1 à 4 atomes de carbone linéaire ou ramifié, trifluorométhyle ou halogène) ou un groupement $-NR_7R_8$ dans lequel $R_7$ et $R_8$ peuvent représenter indépendamment l'un de l'autre un d'hydrogène ou un alkyl inférieur de 1 à 4 atomes de carbone ramifié ou non.
- leurs isomères, diastéréoisomères, énantiomères isolés ou sous forme de mélange,

- leurs sels d'addition à un acide minéral ou organique pharmaceutiquement acceptable.

L'invention s'étend également aux procédés d'obtention des composés de formule générale **(I)** caractérisé en ce que l'on fait réagir un amino alcool de formule générale **(II)** :

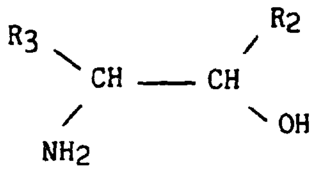

**(II)**

dans laquelle $R_2$ et $R_3$ ont la même signification que dans les composés de formule générale **(I)** en milieu aqueux, en présence d'un acide faible et à une température comprise entre 0 et 20° C avec du glutaraldéhyde puis, avec ou sans isolement intermédiaire avec du cyanure de potassium à une température comprise entre 10° C et 30° C, de manière à obtenir , après isolement et purification par des techniques classiques comme la chromatographie sur colonne de silice et/ou la recristallisation, un composé bicyclique de formule générale **(III)** :

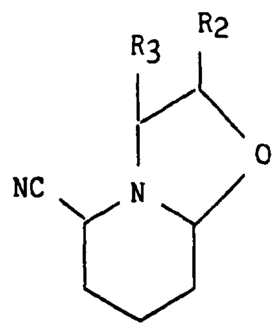

**(III)**

dans laquelle $R_2$ et $R_3$ ont la même signification que dans les composés de formule générale (I), que l'on peut éventuellement faire réagir, en milieu aprotique et à une température comprise entre -78°C et la température ambiante, avec un organolithien de formule générale **(IV)** :

$$R'_4Li \quad (IV)$$

dans laquelle $R'_4$ a la même définition que $R_4$ dans les composés de formule générale **(I)**, avec la réserve que $R'_4$ ne peut pas représenter un atome d'hydrogène, de manière à obtenir une imine de formule générale **(V)** :

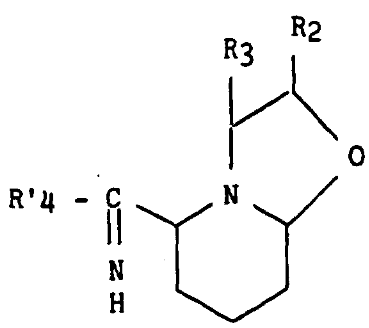

**(V)**

dans laquelle $R_2$ et $R_3$ ont la même signification que dans les composés de formule générale **(I)** et $R'_4$ a la même signification que dans les composés de formule générale **(IV)**, que l'on peut faire réagir, ainsi que les nitriles de formule générale **(III)**, avec un hydrure métallique en milieu aprotique, de manière à obtenir, après isolement et éventuellement purification par des techniques classiques comme la chromatographie sur colonne de silice et/ou la recristallisation, un composé de formule générale **(VI)** :

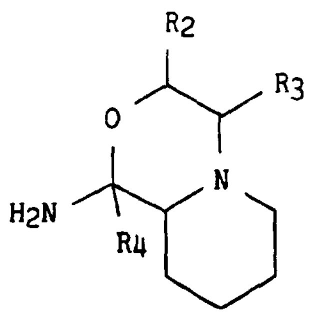

(VI)

dans laquelle $R_2$, $R_3$, $R_4$ ont la même signification que dans les composés de formule générale **(I)** (la configuration des carbones asymétriques des composés de formule générale **(III)** et **(V)** entrainant la configuration des carbones asymétriques des composés de formule générale **(VI)**) que l'on peut faire réagir :

- soit avec un composé halogéné de formule générale **(VII)** :

$$R'_5 - X \qquad (VII)$$

dans laquelle $R'_5$ a la même signification que $R_5$ dans les composés de formule générale **(I)**, avec la réserve que $R'_5$ ne peut pas représenter un atome d'hydrogène et X représente un atome d'halogène, de manière à obtenir, après isolement et éventuellement purification par des techniques classiques comme la chromatographie sur colonne de silice et/ou la recristallisation, un composé de formule générale **(VIII)** :

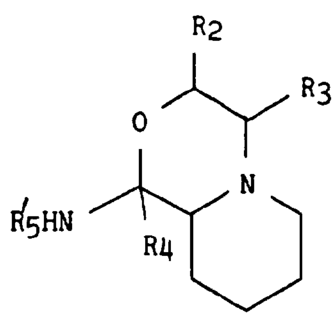

(VIII)

dans laquelle $R_2$, $R_3$ et $R_4$ ont la même signification que dans les composés de formule générale **(I)** et $R'_5$ a la même signification que dans les composés de formule générale (VII), que l'on fait ensuite réagir, éventuellement en présence d'une base, avec un chlorure d'acide de formule générale **(IX)** :

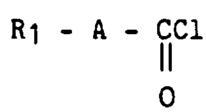

(IX)

dans laquelle $R_1$ et A ont la même signification que dans les composés de formule générale **(I)**, de manière à obtenir, après isolement et purification par des techniques classiques comme la chromatographie sur colonne de silice et/ou la recristallisation, les composés de formule générale **(I)** pour lesquels $R_5$ ne représente pas un atome d'hydrogène,

- soit directement avec un chlorure d'acide, de formule générale **(IX)** de manière à obtenir après isolement et purification par des techniques classiques comme la chromatographie sur colonne de silice et/ou la recristallisation, les composés de formule générale **(I)** pour lesquels $R_5$ est un atome d'hydrogène, que l'on peut ensuite N alkyler selon des techniques classiques, par un composé de formule générale **(VII)**, de manière à obtenir après isolement et éventuellement purification par des techniques classiques comme la chromatographie sur colonne de silice et/ou la recristallisation, les composés de formule générale **(I)** pour lesquels $R_5$ ne représente pas un atome d'hydrogène,

- étant entendu, que les composés de formule générale (I) peuvent, lorsqu'ils ne sont pas diastéréoisomériquement purs être séparés en leurs différents diastéréoisomères et/ou être salifiés par un acide minéral ou organique pharmaceutiquement acceptable.

Les amides de la 1-amino octahydropyrido [2,1-c] [1,4] oxazines selon la présente invention, ainsi que leurs sels d'addition à un acide minéral ou organique pharmaceutiquement acceptable, sont des principes actifs très intéressants pour le traitement des troubles psycho-comportementaux et pouvant être utilisés entre autre dans le traitement des états dépressifs et des psychoses.

Les composés de formule générale **(I)**, ainsi que leurs sels d'addition à un acide minéral ou organique pharmaceutiquement acceptable comme par exemple les acides chlorhydrique, méthanesulfonique, nitrique, maléique, peuvent être présentés sous forme de compositions pharmaceutiques selon des procédés connus, comme par exemple sous forme de comprimés, gélules, dragées, solutions injectables, solutions ou suspensions buvables, émulsions, suppositoires.

Outre les excipients inertes, non toxiques et pharmaceutiquement acceptables, tels par exemple l'eau distillée, le glucose, le lactose l'amidon, le talc, les huiles végétales, l'éthylène glycol etc..., ces compositions peuvent également contenir des agents de préservation.

Les compositions pharmaceutiques ainsi obtenues qui font également partie de l'invention peuvent contenir selon les affections traitées, l'âge et le poids du malade de 1 à 500 mg de principe actif.

Les exemples suivants illustrent l'invention et ne la limitent en aucune manière.

**EXEMPLE 1 : N-(4-PHENYL OCTAHYDROPYRIDO [2,1c] [1,4] OXAZIN-1-YL) 3,4,5-TRIMETHOXY BENZAMIDE (1R) (4R) (9aS)**

**Stade I : 2-amino 2-phényl éthanol (2R)**

Dans un réacteur sous atmosphère d'azote charger 2,5 litres de THF anhydre puis, par petites portions, 38 g (1 mole) d'hydrure de lithium et d'aluminium.

La suspension est chauffée à 60° C puis 100 g (0,66 moles) de 2-phényl glycine (2R) (-) sont additionnés par petites portions. Le milieu réactionnel est ensuite maintenu au reflux pendant 2 h 30 puis refroidi à 5° C.

L'excès d'hydrure est détruit par addition goutte à goutte de 38 cm3 d'une solution aqueuse de soude à 15 %, et finalement 76 cm3 d'eau.

Les sels sont éliminés par filtration, lavés 3 fois par 200 cm3 de THF puis les filtrats réunis sont concentrés sous pression réduite.

Le solide orangé obtenu est séché sous vide, lavé à l'éther éthylique puis recristallisé dans du toluène.

On obtient ainsi 72,6 g (80 %) de 2-amino 2-phényl éthanol (2R) sous forme de cristaux blancs.

Point de fusion : 78° C

Pouvoir rotatoire :

$$\left[\alpha\right]_{D}^{20} = -26,5° \ (CH_3OH, \ C = 0,7)$$

**Stade II : 3-phényl 5-cyano oxazolidino [3,2-a] pipéridine (3R) (5S) (8aR)**

Dans un réacteur charger 1 litre d'eau puis 10 g (0,073 moles) de 2-amino 2-phényl éthanol (2R) et 40 g (0,208 moles) d'acide citrique. Agiter jusqu'à dissolution puis refroidir à 0,5°c et additionner goutte à goutte en 20 minutes 45 $cm^3$ (0,11 moles) d'une solution aqueuse de glutaraldéhyde. Agiter 30 minutes à 0°C puis supprimer le refroidissement et ajouter une solution de 7,15 g (0,11 moles) de cyanure de potassium dans 20 $cm^3$ d'eau ainsi que 200 $cm^3$ de chlorure de méthylène.

Le milieu réactionnel biphasique est agité 3 heures à température ambiante puis la phase aqueuse neutralisée par addition de bicarbonate de sodium.

Après décantation, la phase aqueuse est extraite au chlorure de méthylène et les phases chlorométhyléniques regroupées sont séchées sur sulfate de sodium et concentrées sous pression partielle jusqu'à un volume résiduel de 0,5 litre.

Ajouter alors 2 g de bromure de zinc et agiter vigoureusement sous atmosphère d'azote pendant 3 heures (faire attention à la libération éventuelle de cyanure d'hydrogène). Le milieu réactionnel est ensuite concentré jusqu'à un volume résiduel d'environ 150 $cm^3$ puis purifié par chromatographie sur colonne de silice avec pour éluant un mélange hexane/éther éthylique 2/1. Le produit obtenu est finalement recristallisé dans de l'hexane.

On obtient ainsi 13,9 g (83 %) de 3-phényl 5-cyano oxazolidino [3,2-a] pipéridine (3R) (5S) (8aR).

Point de fusion : 81° C

Pouvoir rotatoire :

$$[\alpha]_D^{20} = -280° \ (CHCl_3, \ C = 1{,}0)$$

RMN $^1H$ 400 MHz (CDCl3) δ : ppm
1,5 -2(m, 6H); 2,13 (dd, j = 11,5 Hz, J' = 1,5 Hz, 1H);
3,74 (t, J = 7,8 Hz, 1H); 3,85 (bd, J = 7,1 Hz, 1H);
3,90 (t, J = 8,0 Hz, 1H); 4,12 (dd, J = 9,7 Hz, J' = 2,8 Hz, 1H);
4,25 (t, J = 7,9 Hz, 1H); 7,4 (m, 5H)
RMN $^{13}C$ δ : ppm
19,3; 28,0; 30,0; 47,4; 63,9; 73,0; 89,9; 116; 128,2; 128,6; 129,0; 137,4

**Stade III : 1-amino 4-phényl octahydropyrido [2,1-c] [1,4]oxazine (1R) (4R) (9aS)**

Dans un réacteur sous atmosphère d'azote charger 2 g (0,0088 moles) de 3-phényl 5-cyano oxazolidino [3,2-a] pipéridine (3R) (5S) (8aR) et 150 $cm^3$ d'hexane distillé. Agiter à 20° C jusqu'à dissolution puis additionner lentement 27 $cm^3$ (0,027 moles) d'une solution 1M d'hydrure de diisobutylaluminium dans l'hexane.

Le milieu réactionnel est agité 4 heures à 20° C puis hydrolysé avec une solution aqueuse à 15 % de soude jusqu'à formation d'un précipité blanc.

La phase solide est extraite plusieurs fois à chaud par du chlorure de méthylène.

Les phases chlorométhyléniques regroupées sont séchées sur sulfate de magnésium puis concentrées sous pression réduite. L'huile incolore obtenue est purifiée par chromatographie sur colonne de silice.

On obtient ainsi 1,1 g (54 %) de 1-amino 4-phényl octahydropyrido [2,1-c] [1,4]oxazine (1R) (4R) (9aS) sous forme de cristaux blancs.
RMN $^1H$ 400 MHz ($CDCl_3$) δ : ppm

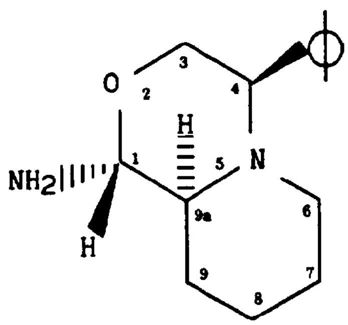

1,3; 1,8; 2,0 3 multiplets, pipéridine
2,7; doublet detriplé, $H_6$ équatorial
3,25; 3,55; 3,70 doublet de doublet, triplet, doublet de doublet, $H_3$; $H_4$
4,00 doublet $H_1$
7,1; 7,3 multiplet phényl
RMN $^{13}C$ ($CDCl_3$) δ : ppm
24,6; 25,5; 28,5; 52,5 ($CH_2$)
67,4; 67,7; 86,5 (CH)
71,5 ($CH_2$)
126,6; 127,9; 128,6 (CH)
130,2 ($C_q$)

**Stade IV : N-(4-phényl octahydropyrido [2,1-c] [1,4] oxazin-1-yl) 3,4,5-triméthoxy benzamide (1R) (4R) (9aS)**

Dans un réacteur sous atmosphère d'azote charger 1,4 g (0,006 moles) de chlorure de 3,4,5-triméthoxy benzoyle et 20 $cm^3$ de dichlorométhane puis additionner la solution préalablement préparée de 1,1 g (0,0047 moles) de 1-amino 4-phényl octahydropyrido [2,1-c] [1,4] oxazine (1R) (4R) (9aS) dans 20 $cm^3$ de dichlorométhane.

Ajouter ensuite 3 $cm^3$ d'une solution aqueuse à 15 % de soude puis agiter 3 heures à 20° C.

Après addition d'eau, extraction au dichlorométhane, séchage des phases chlorométhyléniques sur sulfate de magnésium et concentration sous pression réduite, on obtient 2,3 g de produit brut qui est purifié par flasch chromatographie sur colonne de silice Eluant hexane/éther diéthylique 50/50.

On obtient ainsi 1,3 g (65 %) de N-(4-phényl octahydropyrido [2,1-c] [1,4] oxazin-1-yl) 3,4,5-triméthoxy benzamide sous forme de cristaux blancs.

Point de fusion : 126-128° C (point de fusion du chlorhydrate 270-280° C)

Pouvoir rotatoire :

$$\left[ \alpha \right]_D^{20} = -58,9° \ (CHCl_3, \ C = 0,47)$$

RMN $^1H$ ($CDCl_3$) $\delta$ : ppm

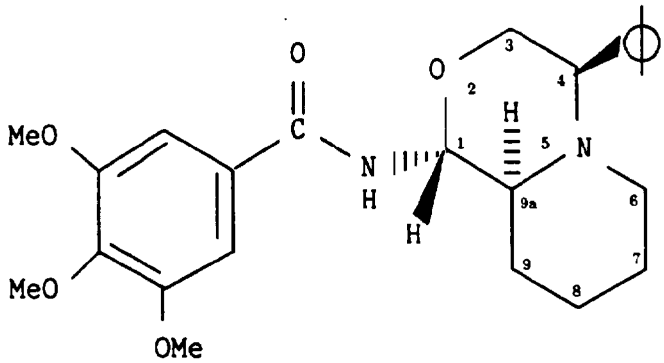

0,9; 1,3; 1,5; 1,9 4 multiplets pipéridine
2,38 triplet $H_{9a}$ axial
2,80 doublet $H_6$ équatorial
3,4; 3,75; 3,8 doublet de doublet, triplet, doublet de doublet $H_3$, $H_4$
3,85 singulet large 3X(OMe)
5,50 triplet $H_1$
7,1 singulet; 7,3 singulet large Noyau aromatique
RMN $^{13}C$ (CDCl3) $\delta$ : ppm
24,4; 25,3; 27,3; 52,5 (CH2)
56,2; 60,7 (CH3)
65,0; 67,2; 81,1 (CH)
72,1 (CH2)
104,9; 127,6; 127,9; 128,6 (CH)
136,6; 153,1; 166,7 (Cq)

| Microanalyse : | | | |
|---|---|---|---|
| calculé<br>Mesuré | C (67,58),<br>C(67,71), | H(7,10),<br>H(6,97), | N(6,57)<br>N(6,29) |

**EXEMPLE 2 : N-(4-ETHYL OCTAHYDROPYRIDO [2,1-c] [1,4] OXAZIN-1-YL) 3,4,5-TRIMETHOXY BENZAMIDE (1R) (4R) (9aS)**

En procédant comme dans l'exemple 1 mais en remplaçant le 2-amino 2-phényl éthanol par le R(-) 2-aminobutanol, on obtient le N-(4-éthyl octahydropyrido [2,1-c] [1,4] oxazin-1-yl) 3,4,5-triméthoxy benzamide (1R) (4R) (9aS)

Point de fusion : 180° C

Pouvoir rotatoire :

$$\left[ \alpha \right]_D^{20} = -67,2° \ (MeOH, \ C = 6,7 \ mg/ml)$$

RMN $^1H$ ($CDCL_3$) $\delta$ : ppm

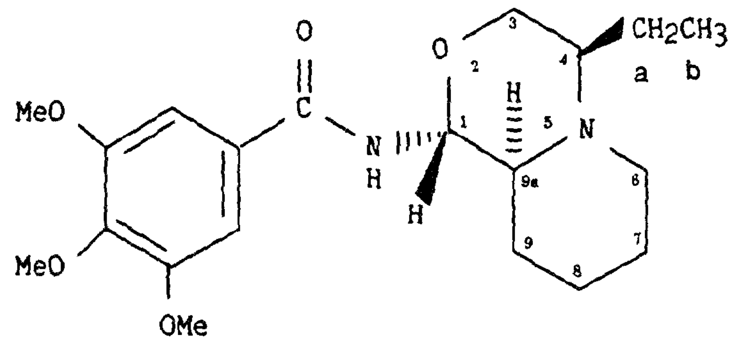

0,85; triplet; 3H; $CH_{3b}$
1,3; 1,6; 1,8; multiplets; pipéridine et $CH_{2a}$
2,05; triplet $H_{9a}$
2,2; multiplet; $H_4$
3,35; doublet; $H_6$ équatorial
3,6; triplet; $H_{3a}$
3,8; multiplet 10H; 3 méthoxy et $H_3$ équatorial
5,3 triplets $H_1$
6,7 doublet NH
7,0; 2H; aromatiques
RMN $^{13}C$ ($CDCl_3$) δ : ppm
10,2 ($CH_3$)
21,8; 24,4; 25,9; 27,7; 51 ($CH_2$)
56,8; 61,3 ($CH_3O$)
61,8; 65,9 (CH)
70,2 ($CH_2$)
81 (CH)
105 (Cq)
129,5 (CH aromatique)
153,6 (Cq aromatique)
167 (C = O)

**EXEMPLES 3 A 6**

En procédant de la même façon que dans l'exemple 1, mais en remplaçant dans le stade I la phénylglycine par :

- la (L) phénylalanine, on obtient le N-(4-benzyl octahydropyrido [2,1-c] [1,4] oxazin-1-yl) 3,4,5-triméthoxy benzamide.
  Point de fusion : 214° C
  Pouvoir rotatoire :

$$[\alpha]_D^{20} = -49,2° \ (CHCl_3, \ C = 7,1 \ mg/ml)$$

  RMN $^1H$ ($CDCL_3$) δ : ppm

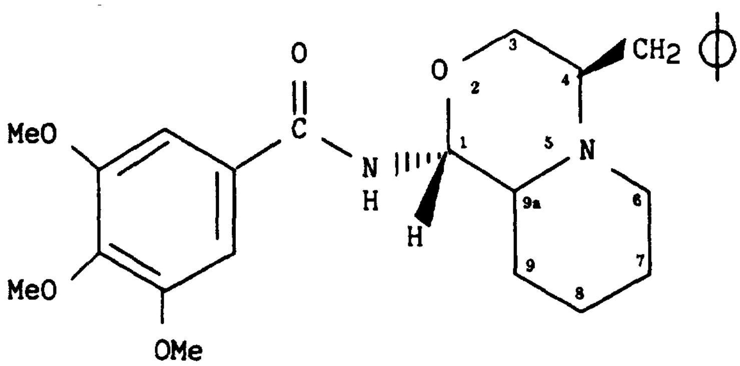

1,1; 1,3; 1,6; 1,75; 4 multiplets ; pipéridine
1,95; ; triplet de doublet; $H_6$ axial
2,05; triplet $H_{9a}$
2,35; doublet de doublets; 1H; $CH_2$ benzylique
2,62; multiplet; $H_4$
3,2; doublet de doublets; 1H; $CH_2$ benzylique
3,5; doublet large; 2H; $H_6$ équatorial $H_3$ axial
3,65; doublet de doublets; $H_3$ équatorial
3,8; 9H; méthoxy
5,3; triplet; $H_1$
6,85; doublet; NH
7; singulet; 2H aromatiques
7,25; multiplet; 5H aromatiques
RMN $^{13}C$ ($CDCl_3$) $\delta$ : ppm
23,7; 26,5; 27,2; 35,6; 51,1 ($CH_2$)
56,2; 58 ($OCH_3$)
61,9; 65,7 (CH)
70 ($CH_2$)
86 (CH)
104 (Cq)
126,5; 128,6; 128,7; 129; 138,3; (CH aromatiques)
153,1 (Cq aromatique)
166 (CO)

- la (L) homophénylalanine, on obtient le N-(4-phénéthyl octahydropyrido [2,1-c] [1,4] ] oxazin-1-yl) 3,4,5-triméthoxy benzamide.

- la (L) leucine, on obtient le N-(4-isopropylméthyl octahydropyrido [2,1-c] [1,4] oxazin-1-yl) 3,4,5-triméthoxy benzamide.

- l'acide 3-cyclohexyl 2-amino propanoique, on obtient le N-(4-cyclohexylméthyl octahydropyrido [2,1-c] [1,4] oxazin-1-yl) 3,4,5-triméthoxy benzamide.

**EXEMPLE 7 : N-(4-PHENYL OCTAHYDROPYRIDO [2,1-c] [1,4] OXAZIN-1-YL) 2,6-DIMETHOXY 3,5-DIBROHO BENZAMIDE (1R) (4R) (9aS)**

En procédant comme dans l'exemple 1 mais en remplaçant dans le stade IV, le chlorure de 3,4,5-triméthoxy benzoyle par le chlorure de 2,6-diméthoxy 3,5-dibromo benzoyle, on obtient le N-(4-phényl octahydropyrido [2,1-c] [1,4] oxazin-1-yl) 2,6-diméthoxy 3,5-dibromo benzamide (1R) (4R) (9aS)
Pouvoir rotatoire :

$$\left[\alpha\right]_D^{20} = -59,5° \ (CHCl_3,\ C = 0,38)$$

RMN $^1H$ ($CDCL_3$) $\delta$ : ppm

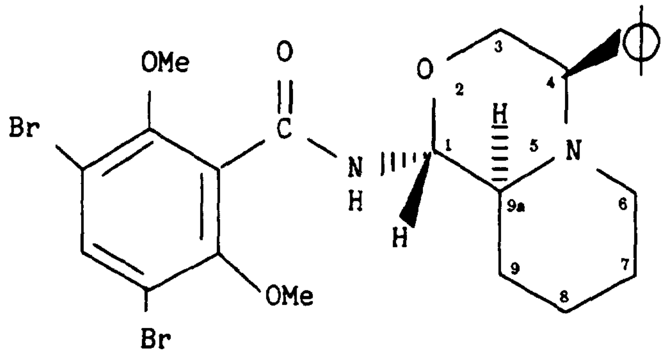

1,3 - 1,9; multiplet 7H, pipéridine
2,0; triplet de doublet, $H_{9a}$ axial
2,70; doublet large, $H_6$ équatorial
3,25; 3,70; 3,80 doublet de doublet, doublet de doublet, triplet $H_3$, $H_4$
3,85 singulet, 6H, 2X($OCH_3$)
5,3 triplet , $H_1$
6,2 doublet, NH - CO
7,3 multiplet 5H, aromatiques
7,8 singulet 1H, aromatique
RMN $^{13}C$ ($CDCl_3$) δ : ppm
24,4; 25,4; 26,9; 52,4; 72,1 (CH2)
62,7 $(-OMe)_2$
65,2; 67,2; 80,3 (CH)
113,0; 127,8; 128,2; 128,6; 137,3 (CH aromatiques)
139,1; 155,0; 164,0 (Cq)

| Microanalyse : | | | |
|---|---|---|---|
| calculé<br>Mesuré | C(49,84),<br>C(49,70), | H(4,73),<br>H(4,82), | N(5,05)<br>N(5,08) |

**EXEMPLE 8 : N-(4-ETHYL OCTAHYDROPYRIDO [2,1-c] [1,4] OXAZIN-1-YL) 3,4-DICHLORO BENZAMIDE (1R) (4R) (9aS)**

En procédant de la même façon que dans l'exemple 2 mais en remplaçant le chlorure de 3,4,5-triméthoxybenzoyle par le chlorure de 3,4-dichlorobenzoyle on obtient le N-(4-éthyl octahydropyrido [2,1-c] [1,4] oxazin-1-yl) 3,4-dichloro benzamide (1R) (4R) (9aS)
Point de fusion : 214° C

**EXEMPLES 9 A 14**

En procédant de la même façon que dans l'exemple 1 mais en remplaçant dans le stade IV le chlorure de 3,4,5-triméthoxybenzoyle par

- le chlorure de benzoyle on obtient le N-(4-phényl octahydropyrido [2,1-c][1,4] oxazin-1-yl) benzamide (1R) (4R) (9aS)
  Point de fusion du chlorhydrate : 210-220° C

- le chlorure de 2,3-dichloro benzoyle on obtient le N-(4-phényl octahydropyrido [2,1-c][1,4] oxazin-1-yl) 3,4-dichloro benzamide (1R) (4R) (9aS)
  Point de fusion du chlorhydrate : 220-225° C

- le chlorure de 4-méthoxybenzoyle on obtient le N-(4-phényl octahydropyrido [2,1-c][1,4] oxazin-1-yl) 4-méthoxybenzamide (1R) (4R) (9aS)
  Point de fusion : 242° C

Pouvoir rotatoire :

$$\left[\alpha\right]_D^{20} = -61{,}5° \ (CHCl_3,\ 10\ mg/ml)$$

- le chlorure de 2-méthoxy 4-amino 5-chloro benzoyle on obtient le N-(4-phényl octahydropyrido [2,1-c][1,4] oxazin-1-yl) 2-méthoxy 4-amino 5-chloro benzamide (1R) (4R) (9aS)
  Point de fusion : 200-204° C
  Pouvoir rotatoire :

$$\left[\alpha\right]_D^{20} = -46{,}6° \ (CHCl_3,\ 10\ mg/ml)$$

- le chlorure de 3,4-diméthoxybenzoyle on obtient le N-(4-phényl octahydropyrido [2,1-c][1,4] oxazin-1-yl) 3,4-diméthoxybenzamide (1R) (4R) (9aS)
  Point de fusion : 160° C
  Pouvoir rotatoire :

$$\left[\alpha\right]_D^{20} = -55{,}9° \ (CHCl_3,\ 10\ mg/ml)$$

- le chlorure de 4-trifluorométhyl benzoyle on obtient le N-(4-phényl octahydropyrido [2,1-c][1,4] oxazin-1-yl) 4-trifluorométhyl benzamide (1R) (4R) (9aS)
  Point de fusion : > 270° C
  Pouvoir rotatoire :

$$\left[\alpha\right]_D^{20} = -59{,}5° \ (CHCl_3,\ 10\ mg/ml)$$

**EXEMPLE 15 : N-(4-PHENYL OCTAHYDROPYRIDO [2,1-c][1,4] OXAZIN-1-YL) 3,4,5-TRIMETHOXY BENZAMIDE (1S) (4S) (9aR)**

En procédant comme dans l'exemple 1 mais en remplaçant dans le stade I, la 2-phényl glycine (2R) (-) par la 2-phényl glycine (2S) (+) on obtient le N-(4-phenyl octahydropyrido [2,1-c][1,4] oxazin-1-yl) 3,4,5-trimethoxy benzamide (1S) (4S) (9aR)

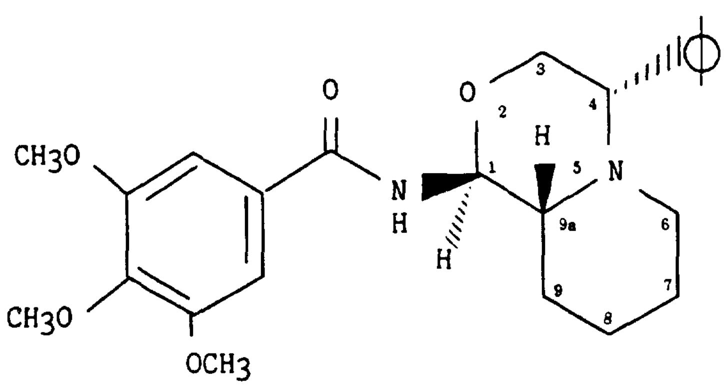

Pouvoir rotatoire :

$$\left[\alpha\right]_D^{20} = +56{,}7° \ (CHCl_3,\ C = 0{,}58)$$

Point de fusion du chlorhydrate : 195 - 200° C

**EXEMPLE 16 : N-(4-METHYL 3-PHENYL OCTAHYDROPYRIDO [2,1-c] [1,4] OXAZIN-1-YL) 3,4,5-TRIMETHOXY BENZAMIDE (1S) (3S) (4R) (9aS)**

**Stade I : 1-amino 3-phényl 4-méthyl octahydropyrido [2,1-c][1,4] oxazine (3S) (4R)**

Ce composé est préparé à partir de 2-phényl 3-méthyl 5-cyano oxazolidino [3,2-a] pipéridine (2S) (3R) (5S) (8aR) par réduction avec de l'hydrure de diisobutylaluminium comme dans le stade III de l'exemple 1.

On obtient la 1-amino 3-phényl 4-méthyl octahydropyrido [2,1-c][1,4] oxazine (3S) (4R), sous forme d'un mélange de deux épimères engagé tel quel dans la suite de la synthèse.

**Stade II : N-(4-méthyl 3-phényl octahydropyrido [2,1-c][1,4] oxazin-1-yl) 3,4,5-triméthozybenzamide (1R) (3S) (4R) (9aS)**

La 1-amino 3-phényl 4-méthyl octahydropyrido [2,1-c][1,4] oxazine (3S) (4R) est traitée par le chlorure de 3,4,5-triméthoxybenzoyle comme dans le stade IV de l'exemple 1.

On obtient un mélange d'épimères séparés par Flasch chromatographie sur colonne de silice (Eluant $CH_2Cl_2$ 98%, $CH_3OH$ 2 %)

L'isomère majoritaire (75 %) est le N-(4-méthyl 3-phényl octahydropyrido [2,1-c][1,4] oxazin-1-yl) 3,4,5-triméthoxy benzamide (1R) (3S) (4R) (9aS)

Point de fusion : 114° C

Pouvoir rotatoire :

$$\left[\alpha\right]_D^{20} = -6° \ (CHCl_3,\ C = 0{,}45)$$

RMN $^1H$ ($CDCl_3$) $\delta$ : ppm

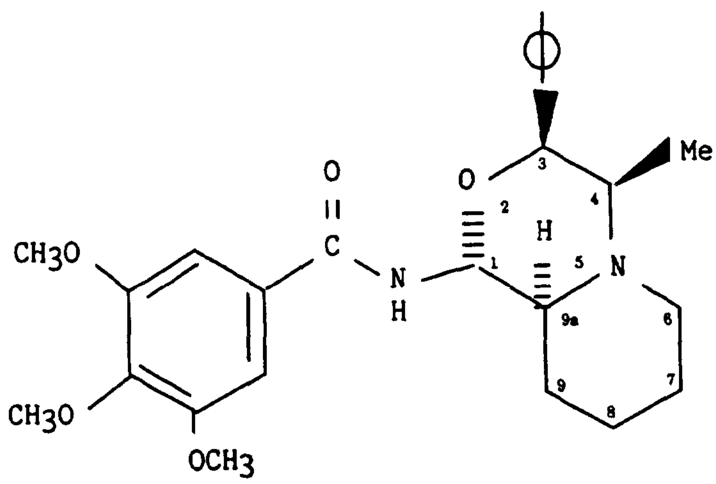

0,80; 3H; $CH_3$ en $C_4$, doublet
1,15 - 1,60; 6H, pipéridine
1,80; 1H; $H_6$ axial, triplet de doublet
2,60; 1H; $H_4$, multiplet
2,80; 1H; $H_{9a}$, quadriplet de doublet
2,90; 1H; $H_6$ équatorial, doublet de multiplet

5,2; 1H, $H_3$, doublet
6,6; 1H; $H_1$
7,3 - 7,6 protons aromatiques
RMN $^{13}C$ ($CDCl_3$) $\delta$ ppm
15,7 ($C_{10}$)
23,7; 25,9; 28,6; 52,6 ($CH_2$)
56,4; 60,9 (- $OCH_3$)
59,6; 60,2 (CH)
75,6; 95,2 (CH)
107,3; 127,5; 128,0 (CH aromatiques)
137,7; 152,9; 165,0 (Cq aromatiques)

| Microanalyse : | | | |
|---|---|---|---|
| calculé | C (68,16), | H(7,32), | N(6,36) |
| Mesuré | C(68.20), | H(7,16), | N(6,43) |

**EXEMPLE 17 : N-(1-METHYL 4-PHENYL OCTATHYDROPYRIDO [2,1-c] [1,4] OXAZIN-1-YL) 3,4,5-TRIMETHOXY BENZAMIDE (1R) (4R)**

**Stade I : 1-amino 1-méthyl 4-phényl octahydropyrido [2,1-c] [1,4] oxazine (1R) (4R)**

Dans un réacteur sous atmosphère d'azote ajouter 11,6 $cm^3$ (0,0185 moles) de méthyl lithium (en solution dans l'éther) à une solution de 3,85 g (0,0168 moles) de 3-phényl 5-cyano oxazolidino [3,2-a] pipéridine (3R) (5S) (8aR) dans 50 $cm^3$ d'éther anhydre à - 5° C.

Agiter 2 h 30 à 20° C puis diluer avec une solution aqueuse de chlorure d'ammonium et extraire la phase aqueuse au chlorure de méthylène.

Après séchage et élimination du solvant, on obtient 3,8 g d'une huile jaune qui est redissoute directement dans 40 $cm^3$ de méthanol puis diluée avec 40 $cm^3$ de THF.

Le milieu réactionnel est amené à PH3 par addition d'HCl 1N puis maintenu à ce PH après addition de 1,16 g de cyano borohydrure de sodium.

Après 90 minutes d'agitation au reflux, le milieu est dilué avec une solution aqueuse saturée en chlorure d'ammonium et extrait au chlorure de méthylène.

Le produit brut est purifié par chromatographie sur colonne de silice (éluant : éther éthylique)

On obtient 2,4 g (60 %) de 1-amino 1-méthyl 4-phényl octahydropyrido [2,1-c] [1,4] oxazine (1R) (4R).
Pouvoir rotatoire :

$$\left[\alpha\right]_D^{20} = -99,9° \ (CHCl_3,\ C = 0,46)$$

RMN $^1H$ ($CDCl_3$) $\delta$ : ppm

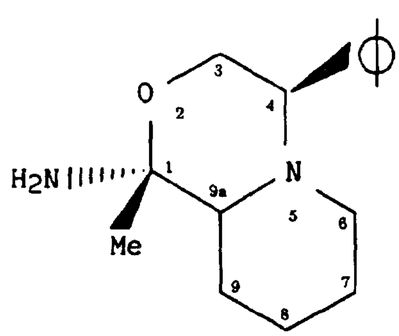

1,2 - 2,2 multiplets, 7H, pipéridine
1,35; singulet, 3H, - $CH_3$
2,1; singulet large, 2H, - $NH_2$
2,2; doublet dédoublé, $H_9a$
2,75; doublet detriplé $H_6$ équatorial

3,2; 3,5; 2 doublets dédoublés, $H_4$ et $H_3$ équatorial
3,8; triplet , $H_3$ axial
7,2 - 7,4; multiplet, 5H, aromatiques
RMN $^{13}C$ ($CDCl_3$) δ : ppm
24,6; 25,4; 27,6; 53,5; 65,4 ($CH_2$)
26,5 ($CH_3$)
68,5; 69,1 (CH)
84,5 (Cq)
127,5; 127,9; 128,5; 140,0 (aromatique)

**Stade II : N-(1-methyl 4-phényl octahydropyrido [2,1-c] [1,4] oxazin-1-yl) 3,4,5-triméthoxy benzamide (1R) (4R)**

En procédant comme dans le stade IV de l'exemple 1 mais en remplaçant la 1-amino 4-phényl octahydropyrido [2,1-c] [1,4] oxazine (1R) (4R) (9aS) par la 1-amino 1-méthyl 4-phényl octahydropyrido [2,1-c] [1,4] oxazine (1R) (4R), on obtient le N-(1-méthyl 4-phényl octahydropyrido [2,1-c] [1,4] oxazin-1-yl) 3,4,5 triméthoxy benzamide (1R) (4R)
Pouvoir rotatoire :

$$\left[\alpha\right]_D^{20} = -47,8° \text{ (CHCl3, C = 4,3)}$$

Point de fusion du chlorhydrate : > 200°
RMN $^1H$ ($CDCl_3$) δ : ppm

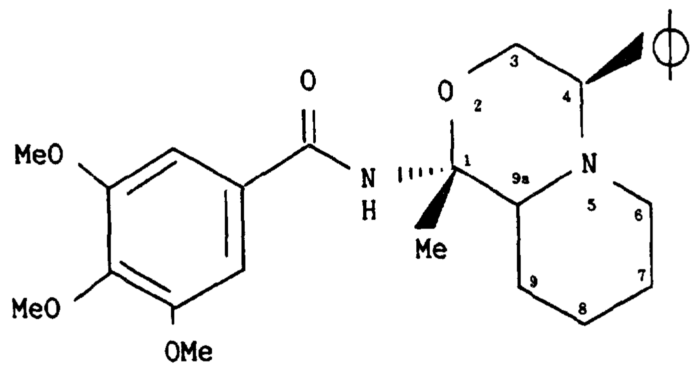

1,3 - 2,0; multiplets; 7H; pipéridine
1,8; singulet; 3H; - $CH_3$
2,3; doublet dédoublé; $H_9a$
2,8; doublet détriplé; $H_6$ équatorial
3,35; 3,6; doublet dédoublé; doublet dédoublé; $H_4$ et $H_3$ équatorial
3,7; triplet $H_3$ axial
3,9; 3,95; 2 singulets; 9H; -$(OCH_3)_3$
7,2; 7,35; 2 singulets; 7H; aromatiques
RMN $^{13}C$ ($CDCl_3$) δ : ppm
24,1; 25,5; 27,1; 52,8; 66,4 ($CH_2$)
23,1 ($CH_3$)
56,3; 60,9 -($OCH_3$)
68,6; 69,4 (CH)
84,0 (Cq)
104,5; 127,6; 127,9; 128,5; 138,9; 153,3 (aromatiques)
166,2 (NH<u>C</u>O)

**EXEMPLE 18 : N-(4-PHENYL OCTAHYDROPYRIDO [2,1-C][1,4] OXAZIN-1-YL) FUR-2-YL CARBOXAMIDE (1R), (4R), (9aS)**

En procédant de la même façon que dans l'exemple I mais en remplaçant dans le stade IV le chlorure de 3,4,5-triméthoxy benzoyle par le chlorure de l'acide furane-2-carboxylique on obtient le N-(4-phényl octahydropyrido [2,1-c]

[1,4] oxazin-1-yl) fur-2-yl carboxamide (1R), (4R), (9aS) avec un rendement de 79 %
Point de fusion du chlorhydrate : 240° C
Pouvoir rotatoire :

$$[\alpha]_D^{20} = -70° \ (CHCl_3,\ 9\ mg/ml)$$

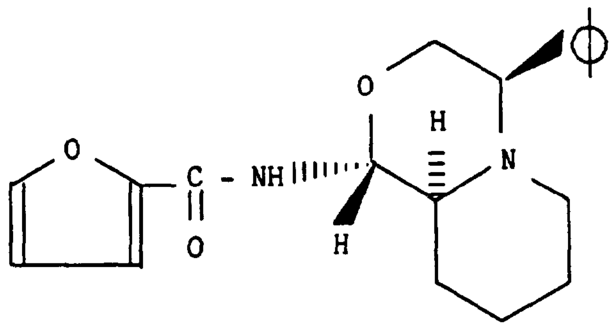

**EXEMPLE 19 : N-(4-PHENYL OCTAHYDROPYRIDO [2,1-C][1,4] OXAZIN-1-YL) INDOL-2-YL CARBOXAMIDE (1R), (4R), (9aS)**

En procédant de la même façon que dans l'exemple I, mais en remplaçant dans le stade IV le chlorure de 3,4,5-triméthoxybenzoyle par le chlorure de l'acide indole-2-carboxylique, on obtient le N-(4-phényl octahydropyrido [2,1-c] [1,4] oxazin-1-yl) indol-2-yl carboxamide (1R), (4R), (9aS)
RMN $^1H$ ($CDCl_3$) $\delta$ : ppm

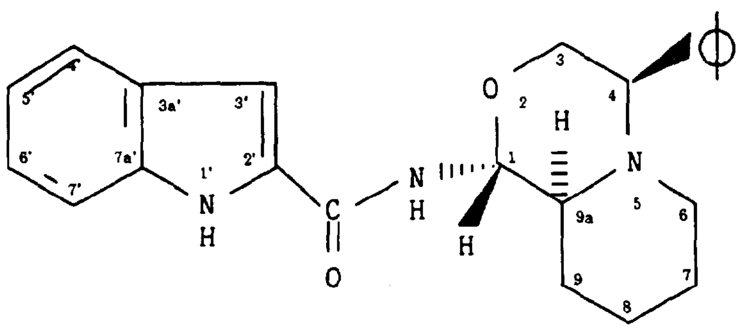

1 - 2; massif; 6H
2,18; doublet de triplet; $H_6$ axial
2,75; doublet élargi; $H_6$ équatorial
3,3; doublet de doublet; $H_4$
3,6 - 3,82; multiplet; 2H; $CH_2$ (3)
5,40; triplet; $H_1$
6,58; doublet élargi; 1H; <u>NH</u>CO
6,97; doublet; $H_{5'}$
7,2 - 7,5; massif; 8H aromatiques
7,68; doublet; $H_{8'}$
9,25; singulet élargi; $NH_{1'}$
RMN $^{13}C$ ($CDCl_3$) $\delta$ : ppm
24,3; 25,37; 27,47 : $C_7$; $C_8$; $C_9$
52,44 : $C_6$
61,35; 65,10 : $C_{9a}$; $C_4$
71,83 : $C_3$
80,51 : $C_1$
103,42; 112,10; 120,74; 122,19; 124,87 : $C_{3'}$; $C_{5'}$; $C_{6'}$; $C_{7'}$; $C_{8'}$

127,79; 128,13; 128,59 : C aromatiques
162 : NHCO

**EXEMPLES 20 A 26**

En procédant de la même façon que dans l'exemple 1, mais en remplacant dans le stade IV le chlorure de 3,4,5-triméthoxybenzoyle par le:

- chlorure de 2,5-diméthoxy cinnamoyle, on obtient le N-(4-phényl octahydropyrido [2,1-c][1,4] oxazin-1-yl) 2,5-diméthoxy cinnamide (1R) (4R) (9aS) :

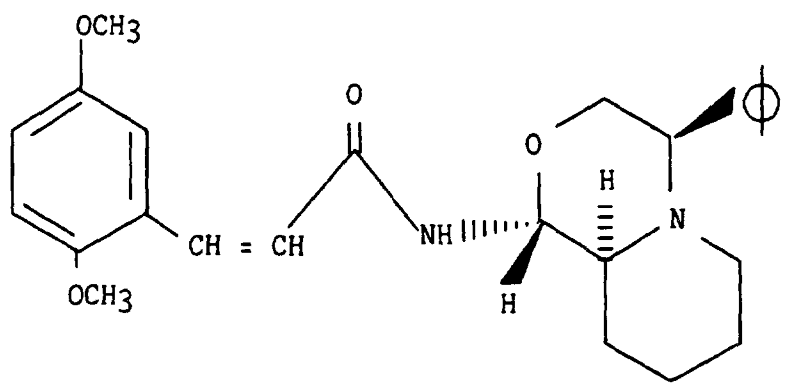

- chlorure de l'acide thiophène 2-carboxylique, on obtient le N-(4-phényl octahydropyrido [2,1-c][1,4] oxazin-1-yl) thiophène-2-yl carboxamide (1R) (4R) (9aS)

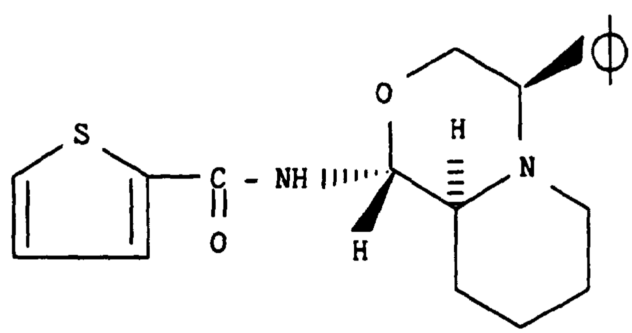

- chlorure de 2-naphtoyle, on obtient le N-(4-phényl octahydropyrido [2,1-c][1,4] oxazin-1-yl) 2-naphtamide (1R) (4R) (9aS)

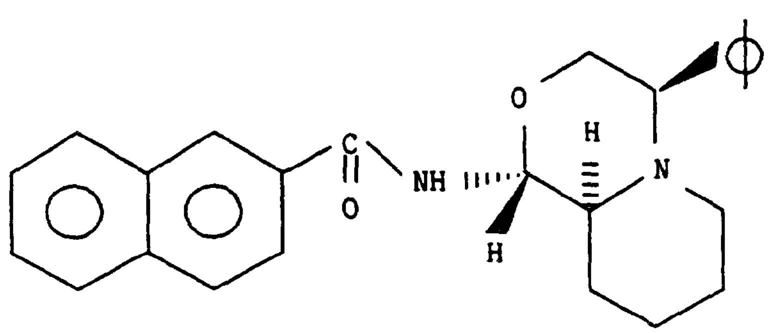

- chlorure de nicotinoyle, on obtient le N-(4-phényl octahydropyrido [2,1-c][1,4] oxazin-1-yl) nicotinamide (1R) (4R) (9aS) :

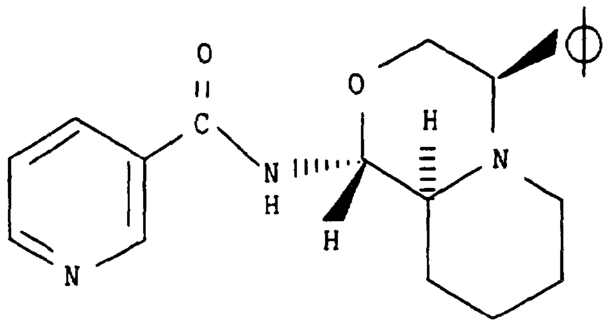

- chlorure de l'acide benzofurane 2-carboxylique, on obtient le N-(4-phényl octahydropyrido [2,1-c][1,4] oxazin-1-yl) benzofurane-2-yl carboxamide :

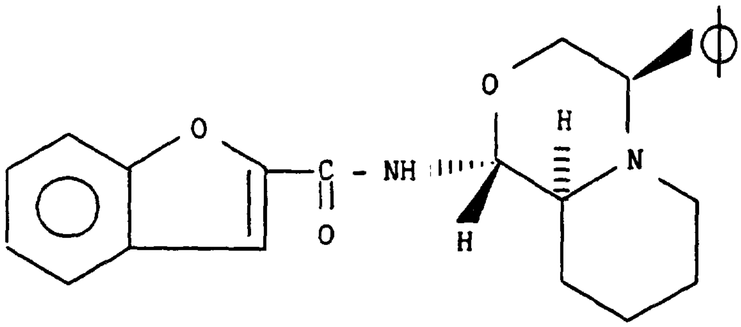

- chlorure de l'acide quinaldique, on obtient le N-(4-phényl octahydropyrido [2,1-c][1,4] oxazin-1-yl) quinoléine-2-yl carboxamide :

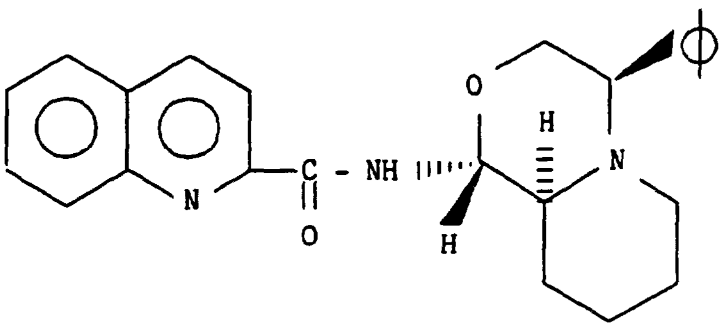

- chlorure de l'acide quinoléine-3-carboxylique, on obtient le N-(4-phényl octahydropyrido [2,1-c][1,4] oxazin-1-yl) quinoléine-3-yl carboxamide :

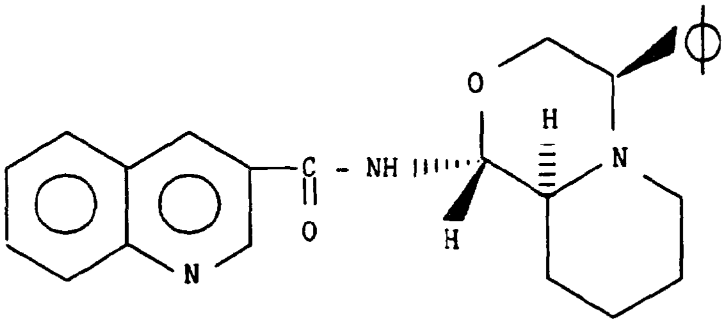

**EXEMPLES 27 A 28**

De la même façon on obtient également le :

- N-(4-phényl octahydropyrido [2,1-c][1,4] oxazin-1-yl) acétamide
- N-(4-phényl octahydropyrido [2,1-c][1,4] oxazin-1-yl) isoquinoléine-3-yl carboxamide

**EXEMPLE 29 : N-(PERHYDRO PYRIDO [2,1-c][1,4] BENZOXAZIN-6-YL) 3,4,5-TRIMETHOXYBENZAMIDE**

En procédant comme dans l'exemple 1 mais en remplaçant dans le stade II le 2-amino 2-phényl éthanol par le 2-amino cyclohexanol on obtient le N-(perhydro pyrido [2,1-c] [1,4] benzoxazin-6-yl) 3,4,5-trimethoxybenzamide.

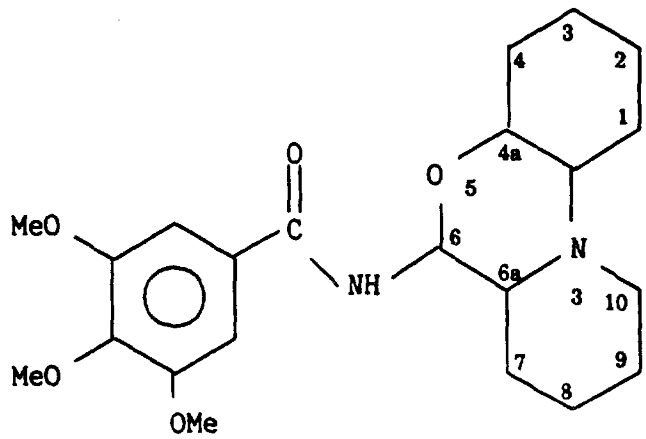

**EXEMPLE 30**

En procédant comme dans l'exemple 2 mais en remplaçant le chlorure de 3,4,5-triméthoxybenzoyle par le chlorure de 3,4-dichlorobenzoyle on obtient le N-(4-éthyl octahydropyrido [2,1-c][1,4] oxazin-1-yl) 3,4-dichloro benzamide (1R) (4R) (9aS)
Point de fusion : 214°C
Pouvoir rotatoire :

$$\left[\alpha\right]_D^{20} = -57,2° \text{ (MeOH, C = 5,5 mg/ml)}$$

RMN $^1H$ ($CDCL_3$) $\delta$ : ppm

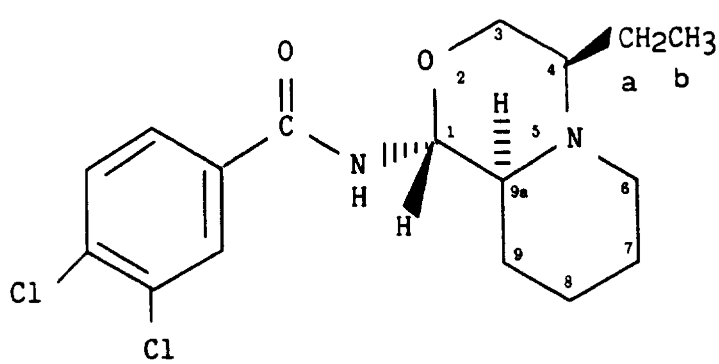

0,85; triplet; 3H; $CH_{3b}$
1,3; 1,6; 1,8; multiplets; protons pipéridine et $CH_{2a}$
1,95 triplet $H_{9a}$

2,15; multiplet $H_4$
3,2; doublet; $H_6$ eq
3,6; triplet; $H_{3a}$
3,8; doublet de doublets $H_3$ eq
5,2 triplet $H_1$
6,5 doublet NH
7,45; 7,6 doublet de doublets; protons aromatiques
7,85; singulet; 1H aromatique
RMN $^{13}C$ ($CDCl_3$) $\delta$ : ppm
10 ($CH_3$)
21,7; 24,2; 25,8; 27,6; 50,8 ($CH_2$)
61,5; 65,6 (CH)
69,9 ($CH_2$)
88,3 (CH)
105 (Cq)
126,6; 129,7; 131 (CH aromatique)
165,3 (C = O)

**EXEMPLE 31 : COMPRIMES DOSES A 25 MG DE N-(4-PHENYL OCTAHYDROPYRIDO [2,1-c] [1,4] OXAZIN-1-YL) 3,4,5-TRIMETHOXY BENZAMIDE (1R) (4R) (9aS)**

| Formule de préparation pour 1000 comprimés | |
|---|---|
| N-(4-phenyl octahydropyrido [2,1-c] [1,4] oxazin-1-yl) 3,4,5-trimethoxy benzamide (1R) (4R) (9aS) | 25 g |
| Amidon de blé | 20 g |
| Amidon de maïs | 20 g |
| Lactose | 60 g |
| Stéarate de magnésium | 1 g |
| Silice | 1 g |
| Hydroxyporpyl cellulose | 2 g |

**EXEMPLE 32 : ETUDE PHARMACOLOGIQUE DES COMPOSES DE L'INVENTION**

A. ANTAGONISME DE L'HYPOTHERMIE INDUITE A L'APOMORPHINE

Ce test est basé sur la capacité qu'ont certains composés psychotropes à antagoniser une hypothermie induite par de l'apomorphine (Puech et Coll Psychopharmacology (1981) 75 p 84-91).

Des souris, randomisées par lot de 6 recoivent une injection sous cutanée de 1 mg/Kg d'apomorphine ce qui provoque une hypothermie, marquée, qui est mesurée 30 minutes après injection.

Les produits à tester sont injectés par voie intra péritonéale 30 minutes avant l'injection d'apomorphine et la température des animaux est mesurée 30 minutes après l'injection d'apomorphine.

Les composés de l'invention antagonisent très significativement l'hypothermie induite par 1 mg/Kg d'apomorphine ce qui est révélateur d'une activité psychotrope.

Ces composés n'inhibent ni l'hypothermie induite par 16 mg/Kg d'apomorphine, ni les stéréotypies et le Climbing induits par 1 mg/Kg d'apomorphine.

B. MISE EN ÉVIDENCE DE L'ACTIVITÉ PSYCHOTROPE SELON LA TECHNIQUE DITE DU "TAIL SUSPENSION TEST".

Ce test est basé sur l'observation du comportement de souris suspendues par la queue.

Les souris, randomisées par lot de 6 sont suspendues par la queue pendant 6 minutes. Les mouvements et les périodes d'immobilité sont enregistrés automatiquement à l'aide d'un appareil permettant de déterminer la durée d'immobilité ainsi que la force des mouvements selon une technique développée par Steru et Coll J. Pharmacol. (1986) 17 348-350 et Prog. Neuro. Psychopharmacol. Biol. Psychiatry (1987) 11 659-671.

Les composés de l'invention comme par exemple le N-(4-phényloctahydro pyrido [2,1-c][1,4] oxazin-1-yl) 3,4,5-triméthoxy benzamide (1R)(4R) (9aS) augmentent fortement la durée d'immobilité ce qui est revélateur d'une activité

"type neuroleptique".

C. ANTAGONISME DE L'HYPERACTIVITE INDUITE CHEZ LE RAT PAR LA D-AMPHETAMINE OU LE METHYLPHENIDATE.

Ce test est basé sur la capacité qu'ont certains composés à inhiber l'hyperactivité induite chez le rat par 1,5 mg/Kg d'amphétamine ou 10 mg/Kg de méthylphénidate.

Des rats Sprague-Dawley pesant de 200 à 250 g reçoivent les composés à tester par voie IP 30 minutes avant l'administration de 1,5 mg/Kg d'amphétamine ou 10 mg/Kg de méthylphénidate.

L'activité locomotrice de ces rats est ensuite mesurée pendant les quatre heures qui suivent l'administration d'amphétamine ou de méthylphenidate.

Les composés de l'invention inhibent très significativement l'hyperactivité induite par la d amphétamine et par le méthylphénidate, ce qui suggère qu'ils possèdent une activité antidopaminergique au niveau central.

**Revendications**

**Revendications pour les Etats contractants suivants : AT, BE, CH, DE, DK, FR, GB, IT, LI, LU, MC, NL, PT, SE**

**1.** 1-acylamino octahydropyrido [2,1-c] [1,4] oxazines de formule générale **(I)** :

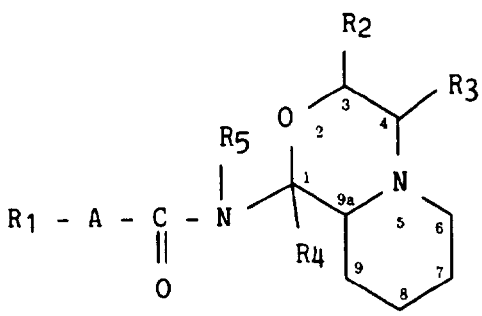

(I)

dans laquelle :

- $R_1$ représente un hydrogène ou un système mono ou bicyclique éventuellement substitué choisi parmi phényl, naphtyl, pyridyl, thiényl, furyl, quinolyl, isoquinolyl, indolyl, indolinyl, perhydroindolyl, benzofuryl,

- A représente une liaison σ ou un alkyl de 1 à 4 atomes de carbone, linéaire ou ramifié, saturé ou insaturé, avec la réserve que lorsque $R_1$ représente un hydrogène, A ne peut représenter une liaison σ,

- $R_2$ et $R_3$ représentent chacun indépendamment l'un de l'autre

  - un hydrogène,
  - un alkyl de 1 à 6 atomes de carbone linéaire ou ramifié et éventuellement substitué,
  - un groupement - $(CH2)_nB$ avec n pouvant prendre les valeurs 0,1,2,3 et B représentant un système monocyclique substitué ou non de 5 à 7 sommets, saturé, insaturé ou aromatique,

  ou R2 et R3 forment ensemble avec les atomes de carbone auxquels ils sont liés un système cyclique saturé éventuellement substitué de 5 à 7 sommets,

- $R_4$ et $R_5$ représentent chacun indépendamment l'un de l'autre un hydrogène, un alkyl de 1 à 4 atomes de carbone linéaire ou ramifié ou un groupement - $(CH_2)_nB$ tel que défini dans la description de $R_2$ et $R_3$,

le terme substitué associé aux systèmes "mono ou bicyclique" signifie que ces cycles peuvent être substitués par un ou des groupements (maximum 4), identiques ou différents, alkyle inférieur de 1 à 4 atomes de carbone ramifié ou non, nitro, amino, alkoxy inférieur de 1 à 4 atomes de carbone ramifié ou non, hydroxy, trifluorométhyle, halogène ou $SO_2R_6$ ,
le terme substitué associé à l'expression alkyle signifie que ce groupement peut être substitué par un ou des

groupements alkoxy inférieur de 1 à 4 atomes de carbone linéaire ou ramifié,

- $R_6$ représente un alkyle inférieur de 1 à 4 atomes de carbone ramifié ou non, un phényl (éventuellement substitué par un ou plusieurs groupements alkyl ou alkoxy inférieur de 1 à 4 atomes de carbone linéaire ou ramifié, trifluorométhyle ou halogène) ou un groupement $-NR_7R_8$ dans lequel $R_7$ et $R_8$ peuvent représenter indépendamment l'un de l'autre un d'hydrogène ou un alkyl inférieur de 1 à 4 atomes de carbone ramifié ou non,
- leurs isomères, diastéréoisomères, énantiomères isolés ou sous forme de mélange,
- leurs sels d'addition à un acide minéral ou organique pharmaceutiquement acceptable.

**2.** Composé selon la revendication 1) qui est le N-(4-phényl octahydropyrido [2,1-c][1,4] oxazin-1-yl) 3,4,5-triméthoxy benzamide ainsi que ses isomères isolés ou sous forme de mélange et leurs sels d'addition à un acide minéral ou organique pharmaceutiquement acceptable.

**3.** Composé selon les revendications 1) et 2) qui est le N-(4-phényl octahydropyrido [2,1-c][1,4] oxazin-1-yl) 3,4,5-triméthoxy benzamide (1R) (4R) (9aS) ainsi que ses sels d'addition à un acide mineral ou organique pharmaceutiquement acceptable

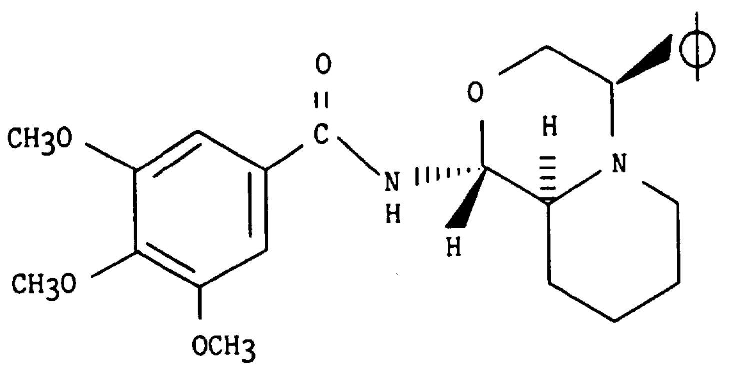

**4.** Composé selon la revendication 1) qui est le N-(4-phényl octahydropyrido [2,1- c][1,4] oxazin-1-yl) 2,6-diméthoxy 3,5-dibromo benzamide ainsi que ses isomères isolés ou sous forme de mélange et leurs sels d'addition à un acide minéral ou organique pharmaceutiquement acceptable.

**5.** Composé selon la revendication 1) qui est le N-(1-méthyl 4-phényl octahydropyrido [2,1-c][1,4] oxazin-1-yl) 3,4,5-triméthoxy benzamide ainsi que ses isomères isolés ou sous forme de mélange et leurs sels d'addition à un acide minéral ou organique pharmaceutiquement acceptable.

**6.** Composé selon la revendication 1) qui est le N-(4-méthyl 3-phényl octahydropyrido [2,1-c][1,4] oxazin-1-yl) 3,4,5-triméthoxy benzamide ainsi que ses isomères isolés ou sous forme de mélange et leurs sels d'addition à un acide minéral ou organique pharmaceutiquement acceptable.

**7.** Procédé d'obtention des composés de formule générale **(I)** selon la revendication 1) caractérisé en ce que l'on fait réagir un amino alcool de formule générale **(II)** :

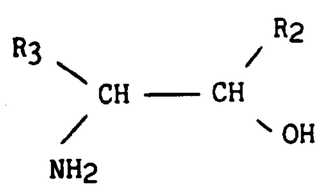

**(II)**

dans laquelle $R_2$ et $R_3$ ont la même signification que dans les composés de formule générale **(I)** en milieu aqueux, en présence d'un acide faible et à une température comprise entre 0 et 20° C avec du glutaraldéhyde puis, avec ou sans isolement intermédiaire avec du cyanure de potassium à une température comprise entre 10° C et 30° C, de manière à obtenir , après isolement et purification par des techniques classiques comme la chromatographie sur colonne de silice et/ou la recristallisation, un composé bicyclique de formule générale **(III)** :

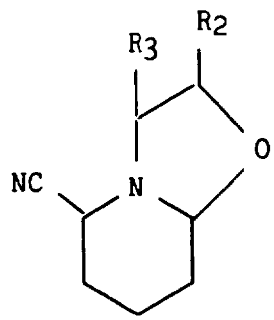

**(III)**

dans laquelle $R_2$ et $R_3$ ont la même signification que dans les composés de formule générale (I), que l'on peut éventuellement faire réagir, en milieu aprotique et à une température comprise entre -78°C et la température ambiante, avec un organolithien de formule générale **(IV)** :

$$R'_4Li \qquad (IV)$$

dans laquelle $R'_4$ a la même définition que $R_4$ dans les composés de formule générale **(I)**, avec la réserve que $R'_4$ ne peut pas représenter un atome d'hydrogène, de manière à obtenir une imine de formule générale **(V)** :

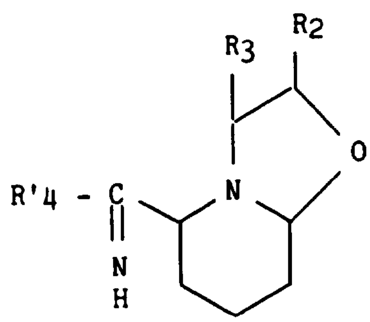

**(V)**

dans laquelle $R_2$ et $R_3$ ont la même signification que dans les composés de formule générale **(I)** et $R'_4$ a la même signification que dans les composés de formule générale **(IV)**, que l'on peut faire réagir, ainsi que les nitriles de formule générale **(III)**, avec un hydrure métallique en milieu aprotique, de manière à obtenir, après isolement et éventuellement purification par des techniques classiques comme la chromatographie sur colonne de silice et/ou la recristallisation, un composé de formule générale **(VI)** :

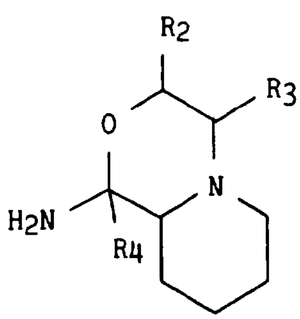

**(VI)**

dans laquelle $R_2$, $R_3$, $R_4$ ont la même signification que dans les composés de formule générale **(I)** (la configuration des carbones asymétriques des composés de formule générale **(III)** et **(V)** entrainant la configuration des carbones asymétriques des composés de formule générale **(VI)**) que l'on peut faire réagir :

- soit avec un composé halogéné de formule générale **(VII)** :

  $$R'_5 - X \qquad (VII)$$

  dans laquelle $R'_5$ a la même signification que $R_5$ dans les composés de formule générale **(I)**, avec la réserve que $R'_5$ ne peut pas représenter un atome d'hydrogène, et X représente un atome d'halogène, de manière à obtenir, après isolement et éventuellement purification par des techniques classiques comme la chromatographie sur colonne de silice et/ou la recristallisation, un composé de formule générale **(VIII)** :

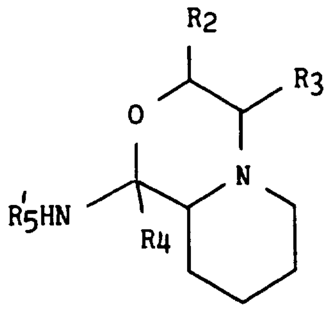

(VIII)

dans laquelle $R_2$, $R_3$ et $R_4$ ont la même signification que dans les composés de formule générale **(I)**, et $R'_5$ a la même signification que dans les composés de formule générale (VII), que l'on fait ensuite réagir, éventuellement en présence d'une base, avec un chlorure d'acide de formule générale **(IX)** :

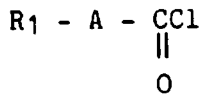

(IX)

dans laquelle $R_1$ et A ont la même signification que dans les composés de formule générale **(I)**, de manière à obtenir, après isolement et purification par des techniques classiques comme la chromatographie sur colonne de silice et/ou la recristallisation, les composés de formule générale **(I)** pour lesquels $R_5$ ne représente pas un atome d'hydrogène,

- soit directement avec un chlorure d'acide, de formule générale **(IX)** de manière à obtenir après isolement et purification par des techniques classiques comme la chromatographie sur colonne de silice et/ou la recristallisation, les composés de formule générale **(I)** pour lesquels $R_5$ est un atome d'hydrogène, que l'on peut ensuite N alkyler selon des techniques classiques, par un composé de formule générale **(VII)**, de manière à obtenir après isolement et éventuellement purification par des techniques classiques comme la chromatographie sur colonne de silice et/ou la recristallisaiton les composés de formule générale **(I)** pour lesquels $R_5$ ne représente pas un atome d'hydrogène,

- étant entendu , que les composés de formule générale (I) peuvent, lorsqu'ils ne sont pas diastéréoisomériquement purs, être séparés en leurs différents diastéréoisomères et/ou être salifiés par un acide minéral ou organique pharmaceutiquement acceptable.

**8.** Composés de formule générale **(VI)** :

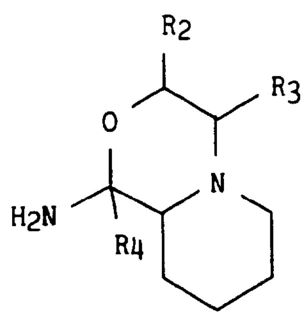

(VI)

dans laquelle $R_2$, $R_3$ et $R_4$ ont la même signification que dans la revendication 1) ainsi que leurs isomères isolés ou sous forme de mélange et leurs sels d'addition à un acide minéral ou organique, utilisables comme intermédiaire de synthèse pour la préparation des composés de formule générale **(I)** selon la revendication 1).

**9.** Compositions pharmaceutiques contenant comme principe actif un composé selon les revendications 1) à 6) seul ou en combinaison avec un ou plusieurs excipients ou véhicules inertes non toxiques pharmaceutiquement acceptable.

**10.** Compositions pharmaceutiques selon la revendication 9) présentées sous une forme convenant notamment pour le traitement des troubles psycho-comportementaux.

**Revendications pour l'Etat contractant suivant : ES**

1. Procédé de préparation des 1-acylamino octahydropyrido [2,1-c] [1,4] oxazines de formule générale **(I)** :

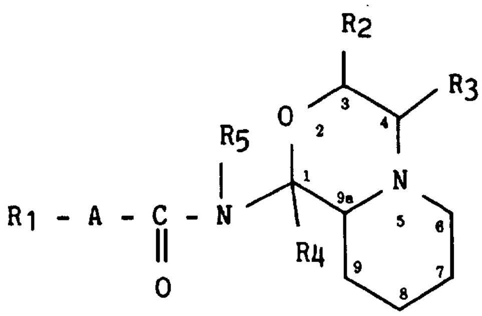

(I)

dans laquelle :

- $R_1$ représente un hydrogène ou un système mono ou bicyclique éventuellement substitué choisi parmi phényl, naphtyl, pyridyl, thiényl, furyl, quinolyl, isoquinolyl, indolyl, indolinyl, perhydroindolyl, benzofuryl,
- A représente une liaison σ ou un alkyl de 1 à 4 atomes de carbone, linéaire ou ramifié, saturé ou insaturé, avec la réserve que lorsque $R_1$ représente un hydrogène, A ne peut représenter une liaison σ,
- $R_2$ et $R_3$ représentent chacun indépendamment l'un de l'autre
  - un hydrogène,
  - un alkyl de 1 à 6 atomes de carbone linéaire ou ramifié et éventuellement substitué,
  - un groupement - $(CH_2)_nB$ avec n pouvant prendre les valeurs 0,1,2,3 et B représentant un système monocyclique substitué ou non de 5 à 7 sommets, saturé, insaturé ou aromatique,

  ou R2 et R3 forment ensemble avec les atomes de carbone auquels ils sont liés un système cyclique saturé éventuellement substitué de 5 à 7 sommets,
- $R_4$ et $R_5$ représentent chacun indépendamment l'un de l'autre un hydrogène, un alkyl de 1 à 4 atomes de carbone linéaire ou ramifié ou un groupement - $(CH_2)_nB$ tel que défini dans la description de $R_2$ et $R_3$,

le terme "substitué" associé aux systèmes "mono ou bicyclique" signifie que ces cycles peuvent être substitués par un ou des groupements (maximum 4), identiques ou différents, alkyle inférieur de 1 à 4 atomes de carbone ramifié ou non, nitro, amino, alkoxy inférieur de 1 à 4 atomes de carbone ramifié ou non, hydroxy, trifluorométhyle, halogène ou $SO_2R_6$ ,
le terme "substitué" associé à l'expression alkyle signifie que ce groupement peut être substitué par un ou des groupements alkoxy inférieur de 1 à 4 atomes de carbone linéaire ou ramifié,

- $R_6$ représente un alkyle inférieur de 1 à 4 atomes de carbone ramifié ou non, un phényl (éventuellement substitué par un ou plusieurs groupements alkyl ou alkoxy inférieur de 1 à 4 atomes de carbone linéaire ou ramifié, trifluorométhyle ou halogène) ou un groupement $-NR_7R_8$ dans lequel $R_7$ et $R_8$ peuvent représenter indépendamment l'un de l'autre un d'hydrogène ou un alkyl inférieur de 1 à 4 atomes de carbone ramifié ou non,
- de leurs isomères, diastéreoisomères, énantiomères isoles ou sous forme de mélange,
- et de leurs sels d'addition à un acide minéral ou organique pharmaceutiquement acceptable,

caractérisé en ce que :
l'on fait réagir un amino alcool de formule générale **(II)** :

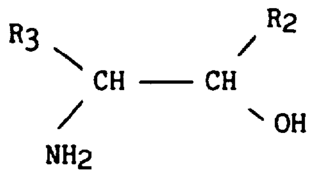

(II)

dans laquelle $R_2$ et $R_3$ ont la même signification que dans les composés de formule générale **(I)** en milieu aqueux, en présence d'un acide faible et à une température comprise entre 0 et 20° C avec du glutaraldéhyde puis, avec ou sans isolement intermédiaire avec du cyanure de potassium à une température comprise entre 10° C et 30° C, de manière à obtenir , après isolement et purification par des techniques classiques comme la chromatographie sur colonne de silice et/ou la recristallisation, un composé bicyclique de formule générale **(III)** :

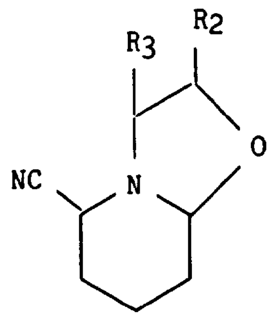

(III)

dans laquelle $R_2$ et $R_3$ ont la même signification que dans les composés de formule générale (I), que l'on peut éventuellement faire réagir, en milieu aprotique et à une température comprise entre -78°C et la température ambiante, avec un organolithien de formule générale **(IV)** :

$$R'_4Li \qquad (IV)$$

dans laquelle $R'_4$ a la même définition que $R_4$ dans les composés de formule générale **(I)**, avec la réserve que $R'_4$ ne peut pas représenter un atome d'hydrogène, de manière à obtenir une imine de formule générale **(V)** :

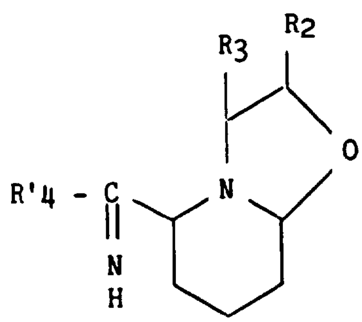

(V)

dans laquelle $R_2$ et $R_3$ ont la même signification que dans les composés de formule générale **(I)** et $R'_4$ a la même signification que dans les composés de formule générale **(IV)**, que l'on peut faire réagir, ainsi que les nitriles de formule générale **(III)**, avec un hydrure métallique en milieu aprotique, de manière à obtenir, après isolement et éventuellement purification par des techniques classiques comme la chromatographie sur colonne de silice et/ou la recristallisation, un composé de formule générale **(VI)** :

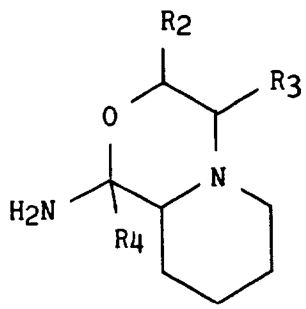

(VI)

dans laquelle $R_2$, $R_3$, $R_4$ ont la même signification que dans les composés de formule générale **(I)** (la configuration des carbones asymétriques des composés de formule générale **(III)** et **(V)** entrainant la configuration des carbones asymétriques des composés de formule générale **(VI)**) que l'on peut faire réagir :

- soit avec un composé halogéné de formule générale **(VII)** :

$$R'_5 - X \qquad (VII)$$

dans laquelle $R'_5$ a la même signification que $R_5$ dans les composés de formule générale **(I)**, avec la réserve que $R'_5$ ne peut pas représenter un atome d'hydrogène, et X représente un atome d'halogène, de manière à obtenir, après isolement et éventuellement purification par des techniques classiques comme la chromatographie sur colonne de silice et/ou la recristallisation, un composé de formule générale **(VIII)** :

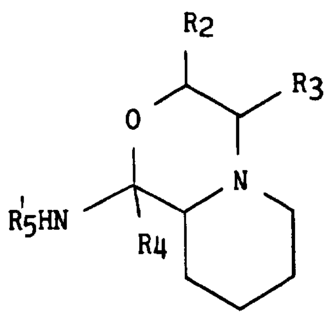

(VIII)

dans laquelle $R_2$, $R_3$ et $R_4$ ont la même signification que dans les composés de formule générale **(I)**, et $R'_5$ a la même signification que dans les composés de formule générale (VII), que l'on fait ensuite réagir, éventuellement en présence d'une base, avec un chlorure d'acide de formule générale **(IX)** :

$$R_1 - A - \underset{\underset{O}{\|}}{C}Cl \qquad (IX)$$

dans laquelle $R_1$ et A ont la même signification que dans les composés de formule générale **(I)**, de manière à obtenir, après isolement et purification par des techniques classiques comme la chromatographie sur colonne de silice et/ou la recristallisation, les composes de formule générale **(I)** pour lesquels $R_5$ ne représente pas un atome d'hydrogène,

- soit directement avec un chlorure d'acide, de formule générale **(IX)** de manière à obtenir après isolement et purification par des techniques classiques comme la chromatographie sur colonne de silice et/ou la recristallisation, les composés de formule générale **(I)** pour lesquels $R_5$ est un atome d'hydrogène, que l'on peut ensuite N alkyler selon des techniques classiques, par un composé de formule générale **(VII)**, de manière à obtenir après Isolement et éventuellement purification par des techniques classiques comme la chromatographie sur colonne de silice et/ou la recristallisation, les composés de formule générale **(I)** pour lesquels $R_5$ ne représente pas un atome d'hydrogène,

- étant entendu que les composés de formule générale (I) peuvent lorsqu'ils ne sont pas diastéréoisomériquement purs être séparés en leurs différents diastéréoisomères et/ou être salifies par un acide minéral ou organique pharmaceutiquement acceptable.

2. Procédé de préparation selon la revendication 1) du composé qui est le N-(4-phényl octahydropyrido [2,1-c][1,4] oxazin-1-yl) 3,4,5-triméthoxy benzamide, ainsi que de ses isomères isolés ou sous forme de mélange et de leurs sels d'addition à un acide mineral ou organique pharmaceutiquement acceptable.

3. Procédé de préparation selon la revendication 1) du composé qui est le N-(4-phényl octahydropyrido [2,1-c][1,4] oxazin-1-yl) 3,4,5-triméthoxy benzamide, ainsi que de ses sels d'addition à un acide minéral ou organique pharmaceutiquement acceptable

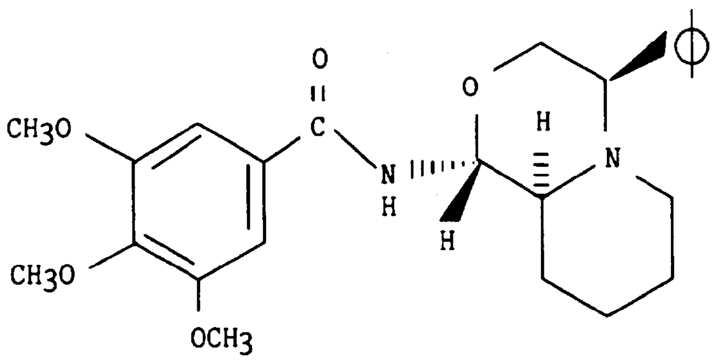

4. Procédé de préparation selon la revendication 1) du composé qui est le N-(4-phényl octahydropyrido [2,1-c][1,4] oxazin-1-yl) 2,6-dimethoxy 3,5-dibromo benzamide, ainsi que de ses isomères isolés ou sous forme de mélange et de leurs sels d'addition à un acide minéral ou organique pharmaceutiquement acceptable.

5. Procédé de préparation selon la revendication 1) du composé qui est le N-(4-méthyl 4-phényl octahydropyrido [2,1-c][1,4] oxazin-1-yl) 3,4,5-triméthoxy benzamide, ainsi que de ses isomères isolés ou sous forme de mélange et de leurs sels d'addition à un acide minéral ou organique pharmaceutiquement acceptable.

6. Procédé de préparation selon la revendication 1) du composé qui est le N-(4-méthyl 3-phényl octahydropyrido [2,1- c] [1,4] oxazin-1-yl) 3,4,5-triméthoxy benzamide, ainsi que de ses isomères isolés ou sous forme de mélange et de leurs sels d'addition à un acide minéral ou organique pharmaceutiquement acceptable.

7. Procédé de préparation des composés de formule générale **(VI)** :

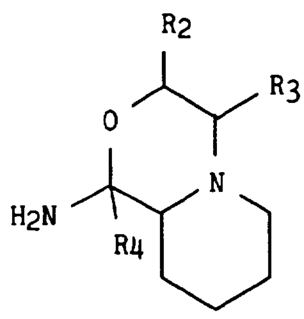

**(VI)**

dans laquelle $R_2$, $R_3$ et $R_4$ ont la même signification que dans la formule (I) de la revendication 1), ainsi que de leurs isomères isolés ou sous forme de mélange, et de leurs sels d'addition à un acide minéral ou organique, utilisables comme intermédiaires de synthèse pour la préparation des composés de formule générale **(I)**, caractérisé en ce que l'on fait réagir un amino alcool de formule générale **(II)** :

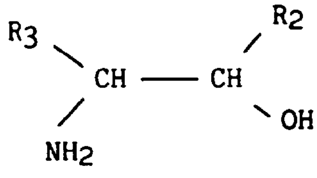

**(II)**

dans laquelle $R_2$ et $R_3$ ont la même signification que dans les composés de formule générale **(I)** en milieu aqueux, en présence d'un acide faible et à une température comprise entre 0 et 20° C avec du glutaraldéhyde puis, avec ou sans isolement intermédiaire avec du cyanure de potassium à une température comprise entre 10° C et 30° C, de manière à obtenir , après isolement et purification par des techniques classiques comme la chromatographie sur colonne de silice et/ou la recristallisation, un composé bicyclique de formule générale **(III)** :

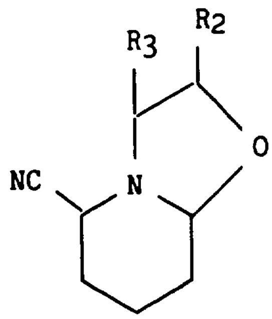

**(III)**

dans laquelle $R_2$ et $R_3$ ont la même signification que dans les composés de formule générale **(I)**, que l'on peut éventuellement faire réagir, en milieu aprotique et à une température comprise entre -78°C et la température ambiante, avec un organolithien de formule générale **(IV)** :

$$R'_4Li \qquad (IV)$$

dans laquelle $R'_4$ a la même définition que $R_4$ dans les composés de formule générale **(I)**, avec la réserve que $R'_4$ ne peut pas représenter un atome d'hydrogène, de manière à obtenir une imine de formule générale **(V)** :

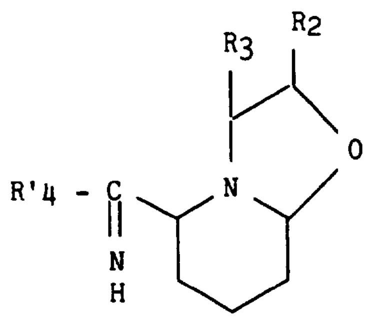

**(V)**

dans laquelle $R_2$ et $R_3$ ont la même signification que dans les composés de formule générale **(I)** et $R'_4$ a la même signification que dans les composés de formule générale **(IV)**, que l'on peut faire réagir, ainsi que les nitriles de formule générale **(III)**, avec un hydrure métallique en milieu aprotique, de manière à obtenir, après isolement et éventuellement purification par des techniques classiques comme la chromatographie sur colonne de silice et/ou la recristallisation, les composés de formule générale **(VI)** :

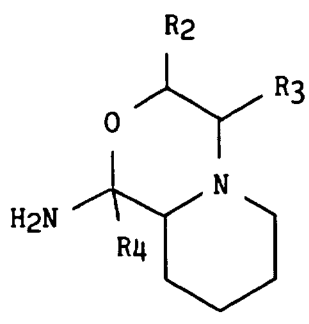

**(VI)**

dans laquelle $R_2$, $R_3$, $R_4$ ont la même signification que dans les composés de formule générale **(I)** (la configuration des carbones asymétriques des composés de formule générale **(III)** et **(V)** entraînant la configuration des carbones asymétriques des composés de formule générale **(VI)**).

**8.** Procédé de préparation d'une composition pharmaceutique contenant comme principe actif un composé préparé selon l'une des revendications 1) à 6) seul ou en combinaison avec un ou plusieurs excipients ou véhicules inertes

non toxiques pharmaceutiquement acceptable.

**9.** Procédé de préparation d'une composition pharmaceutique selon la revendication 8) présentée sous une forme convenant notamment pour le traitement des troubles psycho-comportementaux.

**Revendications pour l'Etat contractant suivant : GR**

**1.** Procédé de préparation des 1-acylamino octahydropyrido [2,1-c] [1,4] oxazines de formule générale (I) :

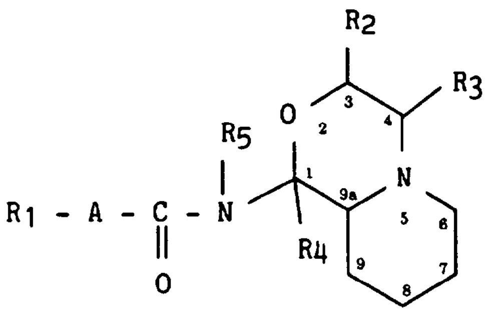

(I)

dans laquelle :

- $R_1$ représente un hydrogène ou un système mono ou bicyclique éventuellement substitué choisi parmi phényl, naphtyl, pyridyl, thiényl, furyl, quinolyl, isoquinolyl, indolyl, indolinyl, perhydroindolyl, benzofuryl,
- A représente une liaison σ ou un alkyl de 1 à 4 atomes de carbone, linéaire ou ramifié, saturé ou insaturé, avec la réserve que lorsque $R_1$ représente un hydrogène, A ne peut représenter une liaison σ,
- $R_2$ et $R_3$ représentent chacun indépendamment l'un de l'autre
  - un hydrogène,
  - un alkyl de 1 à 6 atomes de carbone linéaire ou ramifié et éventuellement substitué,
  - un groupement - $(CH_2)_nB$ avec n pouvant prendre les valeurs 0,1,2,3 et B représentant un système monocyclique substitué ou non de 5 à 7 sommets, saturé, insaturé ou aromatique,

  ou R2 et R3 forment ensemble avec les atomes de carbone auquels ils sont liés un système cyclique saturé éventuellement substitué de 5 à 7 sommets,
- $R_4$ et $R_5$ représentent chacun indépendamment l'un de l'autre un hydrogène, un alkyl de 1 à 4 atomes de carbone linéaire ou ramifié ou un groupement - $(CH_2)_nB$ tel que défini dans la description de $R_2$ et $R_3$,

le terme "substitué" associé aux systèmes "mono ou bicyclique" signifie que ces cycles peuvent être substitués par un ou des groupements (maximum 4), identiques ou différents, alkyle inférieur de 1 à 4 atomes de carbone ramifié ou non, nitro, amino, alkoxy inférieur de 1 à 4 atomes de carbone ramifié ou non, hydroxy, trifluorométhyle, halogène ou $SO_2R_6$ ,
le terme "substitué" associé à l'expression alkyle signifie que ce groupement peut être substitué par un ou des groupements alkoxy inférieur de 1 à 4 atomes de carbone linéaire ou ramifié,

- $R_6$ représente un alkyle inférieur de 1 à 4 atomes de carbone ramifié ou non, un phenyl (éventuellement substitué par un ou plusieurs groupements alkyl ou alkoxy inférieur de 1 à 4 atomes de carbone linéaire ou ramifié, trifluorométhyle ou halogène) ou un groupement $-NR_7R_8$ dans lequel $R_7$ et $R_8$ peuvent représenter independamment l'un de l'autre un d'hydrogène ou un alkyl inférieur de 1 à 4 atomes de carbone ramifie ou non,
- de leurs isomères, diastéreoisomères, énantiomères isolés ou sous forme de mélange,
- et de leurs sels d'addition à un acide minéral ou organique pharmaceutiquement acceptable,

caracterisé en ce que :

l'on fait réagir un amino alcool de formule générale **(II)** :

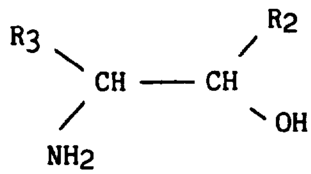

**(II)**

dans laquelle $R_2$ et $R_3$ ont la même signification que dans les composés de formule générale **(I)** en milieu aqueux, en présence d'un acide faible et à une température comprise entre 0 et 20° C avec du glutaraldéhyde puis, avec ou sans isolement intermédiaire avec du cyanure de potassium à une température comprise entre 10° C et 30° C, de manière à obtenir , après isolement et purification par des techniques classiques comme la chromatographie sur colonne de silice et/ou la recristallisation, un composé bicyclique de formule générale **(III)** :

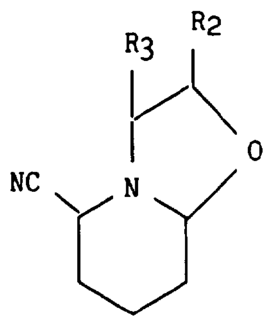

**(III)**

dans laquelle $R_2$ et $R_3$ ont la même signification que dans les composés de formule générale **(I)**, que l'on peut éventuellement faire réagir, en milieu aprotique et à une température comprise entre -78°C et la température ambiante, avec un organolithien de formule générale **(IV)** :

$$R'_4Li \qquad (IV)$$

dans laquelle $R'_4$ a la même définition que $R_4$ dans les composés de formule générale **(I)**, avec la réserve que $R'_4$ ne peut pas représenter un atome d'hydrogène, de manière à obtenir une imine de formule générale **(V)** :

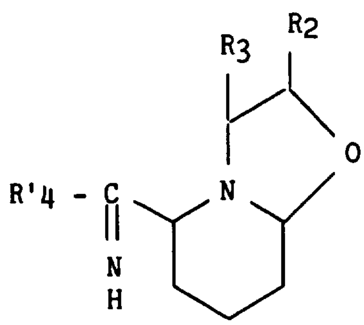

**(V)**

dans laquelle $R_2$ et $R_3$ ont la même signification que dans les composés de formule générale **(I)** et $R'_4$ a la même signification que dans les composés de formule générale **(IV)**, que l'on peut faire réagir, ainsi que les nitriles de formule générale **(III)**, avec un hydrure métallique en milieu aprotique, de manière à obtenir, après isolement et éventuellement purification par des techniques classiques comme la chromatographie sur colonne de silice et/ou la recristallisation, un composé de formule générale **(VI)** :

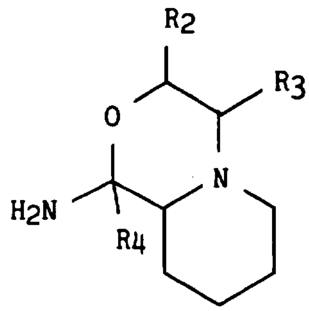

**(VI)**

dans laquelle $R_2$, $R_3$, $R_4$ ont la même signification que dans les composés de formule générale **(I)** (la configuration des carbones asymétriques des composés de formule générale **(III)** et **(V)** entrainant la configuration des carbones asymétriques des composés de formule générale **(VI)**) que l'on peut faire réagir :

- soit avec un composé halogéné de formule générale **(VII)** :

  $$R'_5 - X \qquad \text{(VII)}$$

  dans laquelle $R'_5$ a la même signification que $R_5$ dans les composés de formule générale **(I)**, avec la réserve que $R'_5$ ne peut pas représenter un atome d'hydrogène, et X représente un atome d'halogène, de manière à obtenir, après isolement et éventuellement purification par des techniques classiques comme la chromatographie sur colonne de silice et/ou la recristallisation, un composé de formule générale **(VIII)** :

  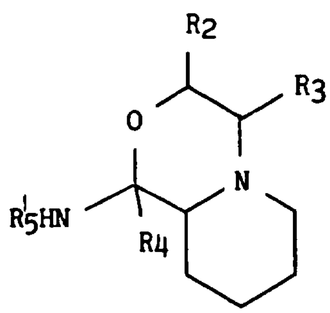

  **(VIII)**

  dans laquelle $R_2$, $R_3$ et $R_4$ ont la même signification que dans les composés de formule générale **(I)**, et $R'_5$ a la même signification que dans les composés de formule générale (VII), que l'on fait ensuite réagir, éventuellement en présence d'une base, avec un chlorure d'acide de formule générale **(IX)** :

  $$R_1 - A - \underset{\underset{O}{\|}}{C}Cl \qquad \textbf{(IX)}$$

  dans laquelle $R_1$ et A ont la même signification que dans les composés de formule générale **(I)**, de manière à obtenir, après isolement et purification par des techniques classiques comme la chromatographie sur colonne de silice et/ou la recristallisation, les composés de formule générale **(I)** pour lesquels $R_5$ ne représente pas un atome d'hydrogène,

- soit directement avec un chlorure d'acide, de formule générale **(IX)** de manière à obtenir après isolement et purification par des techniques classiques comme la chromatographie sur colonne de silice et/ou la recristallisation, les composés de formule générale **(I)** pour lesquels $R_5$ est un atome d'hydrogène, que l'on peut ensuite N alkyler selon des techniques classiques, par un composé de formule générale **(VII)**, de manière à obtenir après isolement et éventuellement purification par des techniques classiques comme la chromatographie sur colonne de silice et/ou la recristallisation, les composés de formule générale **(I)** pour lesquels $R_5$ ne représente pas un atome d'hydrogène,

- étant entendu que les composés de formule générale (I) peuvent lorsqu'ils ne sont pas diastéréoisomériquement purs être séparés en leurs différents diastéréoisomères et/ou être salifiés par un acide minéral ou organique pharmaceutiquement acceptable.

2. Procédé de préparation selon la revendication 1) du composé qui est le N-(4-phényl octahydropyrido [2,1-c] [1,4] oxazin-1-yl) 3,4,5-triméthoxy benzamide, ainsi que de ses isomères isolés ou sous forme de mélange et de leurs sels d'addition à un acide minéral ou organique pharmaceutiquement acceptable.

3. Procedé de préparation selon la revendication 1) du composé qui est le N-(4-phényl octahydropyrido [2,1-c][1,4] oxazin-1-yl) 3,4,5-trimethoxy benzamide (1R) (4R) (9aS), ainsi que de ses sels d'addition à un acide minéral ou organique pharmaceutiquement acceptable

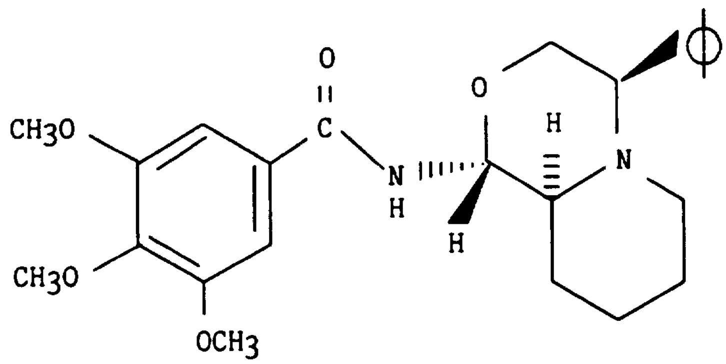

4. Procédé de préparation selon la revendication 1) du composé qui est le N-(4-phényl octahydropyrido [2,1-c][1,4] oxazin-1-yl) 2,6-diméthoxy 3,5-dibromo benzamide, ainsi que de ses isomères isolés ou sous forme de mélange et de leurs sels d'addition à un acide minéral ou organique pharmaceutiquement acceptable.

5. Procédé de préparation selon la revendication 1) du composé qui est le N-(1-méthyl 4-phényl octahydropyrido [2,1-c][1,4] oxazin-1-yl) 3,4,5-triméthoxy benzamide, ainsi que de ses isomères isolés ou sous forme de mélange et de leurs sels d'addition à un acide minéral ou organique pharmaceutiquement acceptable.

6. Procédé de préparation selon la revendication 1) du composé qui est le N-(4-méthyl 3-phényl octahydropyrido [2,1-c][1,4] oxazin-1-yl) 3,4,5-triméthoxy benzamide, ainsi que de ses isomères isoles ou sous forme de mélange et de leurs sels d'addition à un acide minéral ou organique pharmaceutiquement acceptable.

7. Procédé de préparation des composés de formule générale **(VI)** :

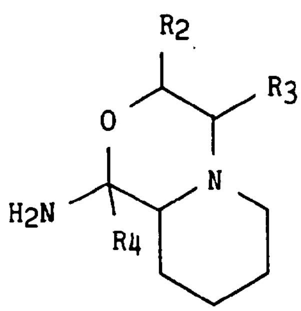

**(VI)**

dans laquelle $R_2$, $R_3$ et $R_4$ ont la même signification que dans la formule (I) de la revendication 1), ainsi que de leurs isomères isolés ou sous forme de mélange, et de leurs sels d'addition à un acide minéral ou organique, utilisables comme intermédiaires de synthèse pour la préparation des composés de formule générale **(I)**, caractérisé en ce que l'on fait réagir un amino alcool de formule générale **(II)** :

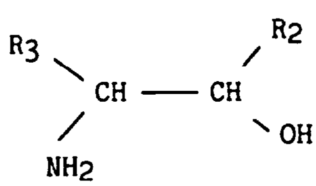

**(II)**

dans laquelle $R_2$ et $R_3$ ont la même signification que dans les composés de formule générale **(I)** en milieu aqueux, en présence d'un acide faible et à une température comprise entre 0 et 20° C avec du glutaradéhyde puis, avec

ou sans isolement intermédiaire avec du cyanure de potassium à une température comprise entre 10° C et 30° C, de manière à obtenir , après isolement et purification par des techniques classiques comme la chromatographie sur colonne de silice et/ou la recristallisation, un composé bicyclique de formule générale **(III)** :

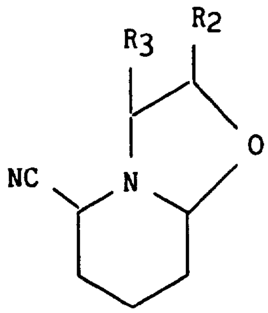

(III)

dans laquelle $R_2$ et $R_3$ ont la même signification que dans les composés de formule générale (I), que l'on peut éventuellement faire réagir, en milieu aprotique et à une température comprise entre -78°C et la température ambiante, avec un organolithien de formule générale **(IV)** :

$$R'_4Li \quad (IV)$$

dans laquelle $R'_4$ a la même définition que $R_4$ dans les composés de formule générale **(I)**, avec la réserve que $R'_4$ ne peut pas représenter un atome d'hydrogène, de manière à obtenir une imine de formule générale **(V)** :

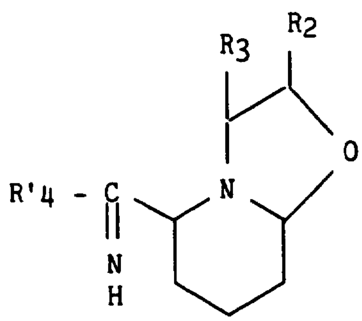

(V)

dans laquelle $R_2$ et $R_3$ ont la même signification que dans les composés de formule générale **(I)** et $R'_4$ a la même signification que dans les composés de formule générale **(IV)**, que l'on peut faire réagir, ainsi que les nitriles de formule générale **(III)**, avec un hydrure métallique en milieu aprotique, de manière à obtenir, après isolement et éventuellement purification par des techniques classiques comme la chromatographie sur colonne de silice et/ou la recristallisation, les composés de formule générale **(VI)** :

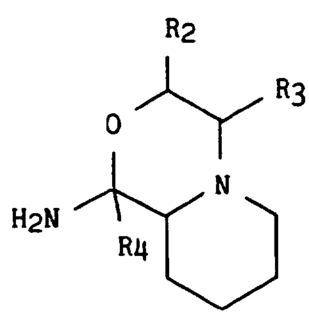

(VI)

dans laquelle $R_2$, $R_3$, $R_4$ ont la même signification que dans les composés de formule générale **(I)** (la configuration des carbones assymétriques des composés de formule générale **(III)** et **(V)** entraînant la configuration des carbones asymétriques des composés de formule générale **(VI)**).

**8.** Procédé de préparation d'une composition pharmaceutique contenant comme principe actif un composé préparé selon l'une des revendications 1) à 6) seul ou en combinaison avec un ou plusieurs excipients ou véhicules inertes non toxiques pharmaceutiquement acceptable.

**9.** Procédé de préparation d'une composition pharmaceutique selon la revendication 8) présentée sous une forme convenant notamment pour le traitement des troubles psycho-comportementaux.

## Patentansprüche

**Patentansprüche für folgende Vertragsstaaten : AT, BE, CH, DE, DK, FR, GB, IT, LI, LU, MC, NL, PT, SE**

**1.** 1-Acylamino-octahydropyrido[2,1-c][1,4]oxazine der allgemeinen Formel (I):

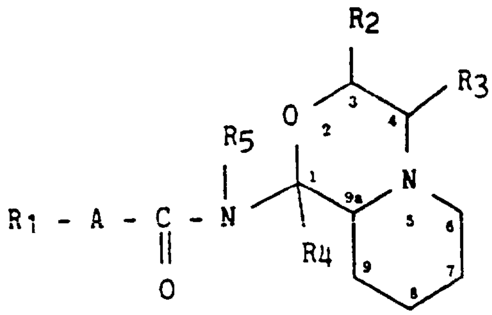

(I)

in der:

- $R_1$ Wasserstoff oder ein gegebenenfalls substituiertes mono- oder bicyclisches System ausgewählt aus Phenyl, Naphthyl, Pyridyl, Thienyl, Furyl, Chinolyl, Isochinolyl, Indolyl, Indolinyl, Perhydroindolyl und Benzofuryl,
- A eine σ-Bindung oder eine geradkettige oder verzweigte, gesättigte oder ungesättigte Alkylgruppe mit 1 bis 4 Kohlenstoffatomen mit der Maßgabe, daß, wenn $R_1$ Wasserstoff darstellt, A keine σ-Bindung bedeutet,
- $R_2$ und $R_3$ jeweils unabhängig voneinander
  - Wasserstoff,
  - eine geradkettige oder verzweigte und gegebenenfalls substituierte Alkylgruppe mit 1 bis 6 Kohlenstoffatomen,
  - eine Gruppe $-(CH_2)_nB$, worin n Werte von 0, 1, 2 und 3 annehmen kann und B ein gegebenenfalls substituiertes, gesättigtes, ungesättigtes oder aromatisches monocyclisches System mit 5 bis 7 Ringgliedern,

  oder $R_2$ und $R_3$ gemeinsam mit den Kohlenstoffatomen, an die sie gebunden sind, ein gesättigtes, gegebenenfalls substituiertes cyclisches System mit 5 bis 7 Ringgliedern,
- $R_4$ und $R_5$ jeweils unabhängig voneinander Wasserstoff, eine geradkettige oder verzweigte Alkylgruppe mit 1 bis 4 Kohlenstoffatomen oder eine Gruppe $-(CH_2)_nB$, wie sie bezüglich $R_2$ und $R_3$ definiert worden ist, bedeuten,

wobei der für die "mono- oder bicyclischen" Systeme verwendete Begriff "substituiert" bedeutet, daß diese Ringe durch eine oder mehrere (maximal 4) gleichartige oder verschiedene, gegebenenfalls verzweigte Niedrigalkylgruppen mit 1 bis 4 Kohlenstoffatomen, Nitrogruppen, Aminogruppen, gegebenenfalls verzweigte Niedrigalkoxygruppen mit 1 bis 4 Kohlenstoffatomen, Hydroxylgruppen, Trifluormethylgruppen, Halogenatome oder $SO_2R_6$-Gruppen substituiert sein können,
der mit dem Ausdruck Alkyl benutzte Begriff "substituiert" bedeutet, daß die Gruppe durch eine oder mehrere geradkettige oder verzweigte Niedrigalkoxygruppen mit 1 bis 4 Kohlenstoffatomen substituiert sein kann,

- worin $R_6$ eine gegebenenfalls verzweigte Niedrigalkylgruppe mit 1 bis 4 Kohlenstoffatomen, eine Phenylgruppe (die gegebenenfalls durch eine oder mehrere geradkettige oder verzweigte Niedrigalkyl- oder Niedrigalkoxygruppen mit 1 bis 4 Kohlenstoffatomen, Trifluormethylgruppen oder Halogenatome substituiert sein kann) oder eine Gruppe $-NR_7R_8$ darstellt, worin $R_7$ und $R_8$ unabhängig voneinander Wasserstoff oder eine gegebenenfalls verzweigte Niedrigalkylgruppe mit 1 bis 4 Kohlenstoffatomen darstellen,
- deren Isomere, Diastereoisomere und Enantiomere in isolierter Form oder in Form einer Mischung,
- und deren Additionssalze mit einer anorganischen oder organischen, pharmazeutisch annehmbaren Säure.

2. Verbindung nach Anspruch 1, nämlich N-(4-Phenyl-octahydropyrido-[2,1-c][1,4]oxazin-1-yl)-3,4,5-trimethoxy-benzamid sowie dessen Isomere in isolierter Form oder in Form einer Mischung und dessen Additionssalze mit einer anorganischen oder organischen, pharmazeutisch annehmbaren Säure.

3. Verbindung nach den Ansprüchen 1 und 2, nämlich N-(4-Phenyl-octahydropyrido[2,1-c][1,4]oxazin-1-yl)-3,4,5-trimethoxy-benzamid (1R) (4R) (9aS) sowie dessen Additionssalze mit einer anorganischen oder organischen, pharmazeutisch annehmbaren Säure:

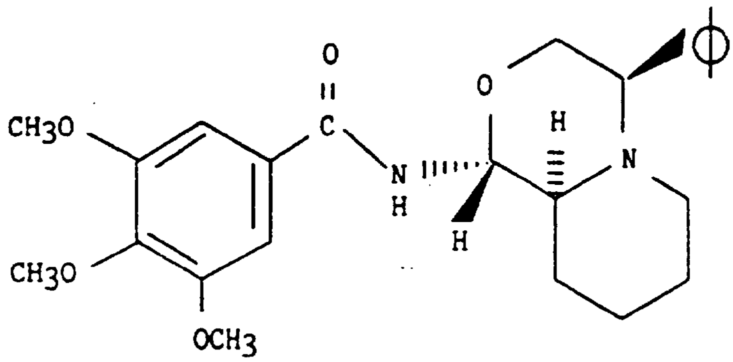

4. Verbindung nach Anspruch 1, nämlich N-(4-Phenyl-octahydropyrido[2,1-c][1,4]oxazin-1-yl)-2,6-dimethoxy-3,5-dibrombenzamid sowie dessen Isomere in isolierter Form oder in Form einer Mischung und dessen Additionssalze mit einer anorganischen oder organischen, pharmazeutisch annehmbaren Säure.

5. Verbindung nach Anspruch 1, nämlich N-(1-Methyl-4-phenyl-octahydropyrido[2,1-c][1,4]oxazin-1-yl)-3,4,5-trimethoxybenzamid sowie dessen Isomere in isolierter Form oder in Form einer Mischung und dessen Additionssalze mit einer anorganischen oder organischen, pharmazeutisch annehmbaren Säure.

6. Verbindung nach Anspruch 1, nämlich N-(4-Methyl-3-phenyl-octahydropyrido[2,1-c][1,4]oxazin-1-yl)-3,4,5-trimethoxybenzamid sowie dessen Isomere in isolierter Form oder in Form einer Mischung und dessen Additionssalze mit einer anorganischen oder organischen, pharmazeutisch annehmbaren Säure.

7. Verfahren zur Herstellung der Verbindungen der allgemeinen Formel (I) nach Anspruch 1, **dadurch gekennzeichnet**, daß man einen Aminoalkohol der allgemeinen Formel (II):

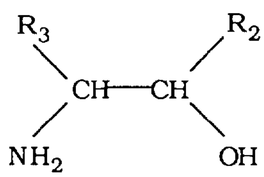

(II)

in der $R_2$ und $R_3$ die bezüglich der Verbindungen der allgemeinen Formel (I) angegebenen Bedeutungen besitzen, in wäßrigem Medium, in Gegenwart einer schwachen Säure und bei einer Temperatur zwischen 0 und 20°C mit Glutaraldehyd und dann mit oder ohne zwischenzeitliche Isolierung mit Kaliumcyanid bei einer Temperatur zwischen 10°C und 30°C umsetzt, so daß man nach der Isolierung und Reinigung mit Hilfe klassischer Verfahrensweisen, wie der Säulenchromatographie über Siliciumdioxid und/oder der Umkristallisation, eine bicyclische Verbindung der allgemeinen Formel (III) erhält:

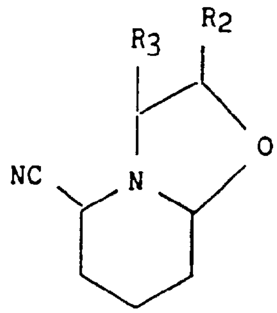

(III)

in der $R_2$ und $R_3$ die bezüglich der Verbindungen der allgemeinen Formel (I) angegebenen Bedeutungen besitzen, welche man gegebenenfalls in aprotischem Medium und bei einer Temperatur zwischen -78°C und Raumtemperatur mit einer lithiumorganischen Verbindung der allgemeinen Formel (IV) umsetzen kann:

$$R'_4Li \qquad (IV)$$

in der $R'_4$ die gleichen Bedeutungen besitzt wie $R_4$ bezüglich der Verbindungen der allgemeinen Formel (I), mit der Maßgabe, daß $R'_4$ kein Wasserstoffatom darstellt, so daß man ein Imin der allgemeinen Formel (V) erhält:

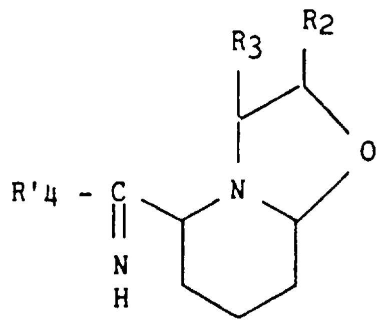

(V)

in der $R_2$ und $R_3$ die bezüglich der Verbindungen der allgemeinen Formel (I) angegebenen Bedeutungen besitzen und $R'_4$ die bezüglich der Verbindungen der allgemeinen Formel (IV) angegebenen Bedeutungen besitzt, welches man ebenso wie die Nitrile der allgemeinen Formel (III) mit einem Metallhydrid in aprotischem Medium umsetzen kann, so daß man nach der Isolierung und der eventuellen Reinigung mit Hilfe klassischer Verfahrensweisen, wie der Säulenchromatographie über Siliciumdioxid und/oder der Umkristallisation, eine Verbindung der allgemeinen Formel (VI) erhält:

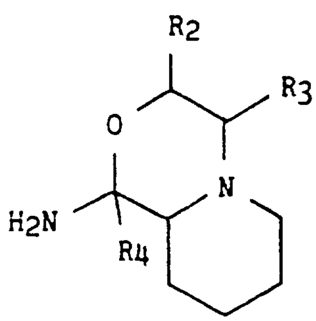

(VI)

in der $R_2$, $R_3$ und $R_4$ die bezüglich der Verbindungen der allgemeinen Formel (I) angegebenen Bedeutungen besitzen (wobei die Konfiguration der asymmetrischen Kohlenstoffatome der Verbindungen der allgemeinen Formel (III) und (V) die Konfiguration der asymmetrischen Kohlenstoffatome der Verbindungen der allgemeinen Formel (VI) bedingt), welche man:

- entweder mit einer Halogenverbindung der allgemeinen Formel (VII):

  $$R'_5\text{-}X \qquad (VII)$$

  in der $R'_5$ die Bedeutungen für $R_5$ bezüglich der Verbindungen der allgemeinen Formel (I) besitzt, mit der Maßgabe, daß $R'_5$ kein Wasserstoffatom darstellt, und X ein Halogenatom bedeutet, umsetzen kann, so daß

man nach der Isolierung und der eventuellen Reinigung mit Hilfe klassischer Verfahrensweisen, wie der Säulenchromatographie über Siliciumdioxid und/oder der Umkristallisation, eine Verbindung der allgemeinen Formel (VIII) erhält:

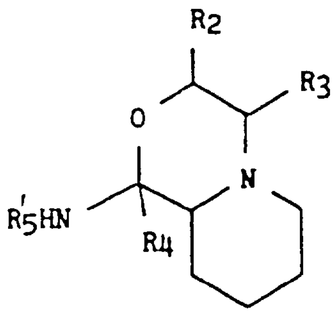

(VIII)

in der $R_2$, $R_3$ und $R_4$ die bezüglich der Verbindungen der allgemeinen Formel (I) angegebenen Bedeutungen besitzen, und $R'_5$ die bezüglich der Verbindungen der allgemeinen Formel (VII) angegebenen Bedeutungen besitzt, welche man anschließend gegebenenfalls in Gegenwart einer Base mit einem Säurechlorid der allgemeinen Formel (IX):

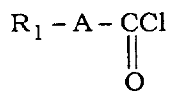

(IX)

in der $R_1$ und A die bezüglich der Verbindungen der allgemeinen Formel (I) angegebenen Bedeutungen besitzen, umsetzt, so daß man nach der Isolierung und der Reinigung mit Hilfe klassischer Verfahrensweisen, wie der Säulenchromatographie über Siliciumdioxid und/oder der Umkristallisation, die Verbindungen der allgemeinen Formel (I) erhält, in der $R_5$ kein Wasserstoffatom darstellt,

- oder direkt mit einem Säurechlorid der allgemeinen Formel (IX) umsetzt, so daß man nach der Isolierung und der Reinigung mit Hilfe klassischer Verfahrensweisen, wie der Säulenchromatographie über Siliciumdioxid und/ oder der Umkristallisation, die Verbindungen der allgemeinen Formel (I) erhält, in der $R_5$ ein Wasserstoffatom darstellt, welche man anschließend mit Hilfe klassischer Verfahrensweisen mit einer Verbindung der allgemeinen Formel (VII) N-alkylieren kann, so daß man nach der Isolierung und der eventuellen Reinigung mit Hilfe klassischer Verfahrensweisen, wie der Säulenchromatographie über Siliciumdioxid und/oder der Umkristallisation, die Verbindungen der allgemeinen Formel (I) erhält, worin $R_5$ kein Wasserstoffatom darstellt,
- mit der Maßgabe, daß die Verbindungen der allgemeinen Formel (I), wenn sie nicht Diastereoisomeren-rein sind, in ihre verschiedenen Diastereoisomere getrennt und/oder mit einer anorganischen oder organischen, pharmazeutisch annehmbaren Säure in ihre Salze überführt werden können.

**8.** Verbindungen der allgemeinen Formel (VI):

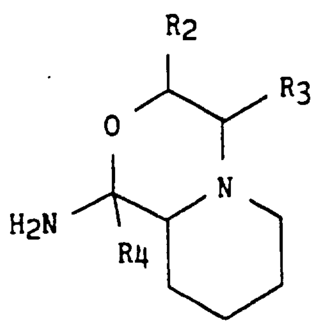

(VI)

in der $R_2$, $R_3$ und $R_4$ die in Anspruch 1 angegebenen Bedeutungen besitzen, sowie deren Isomere in isolierter Form oder in Form einer Mischung und deren Additionssalze mit einer anorganischen oder organischen Säure, als Synthesezwischenprodukt für die Herstellung der Verbindungen der allgemeinen Formel (I) nach Anspruch 1.

**9.** Pharmazeutische Zubereitungen enthaltend als Wirkstoff eine Verbindung nach einem der Ansprüche 1 bis 6 allein oder in Kombination mit einem oder mehreren inerten, nichttoxischen, pharmazeutisch annehmbaren Trägermaterialien oder Bindemitteln.

**10.** Pharmazeutische Zubereitungen nach Anspruch 9 in einer Form, die insbesondere für die Behandlung von Psycho-Verhaltensstörungen geeignet ist.

**Patentansprüche für folgenden Vertragsstaat : ES**

1. Verfahren zur Herstellung der 1-Acylamino-octyhydropyrido[2,1-c][1,4]-oxazine der allgemeinen Formel (I):

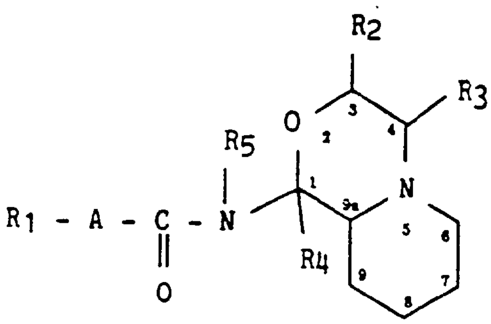

(I)

in der:

- $R_1$ Wasserstoff oder ein gegebenenfalls substituiertes mono- oder bicyclisches System ausgewählt aus Phenyl, Naphthyl, Pyridyl, Thienyl, Furyl, Chinolyl, Isochinolyl, Indolyl, Indolinyl, Perhydroindolyl und Benzofuryl,
- A eine σ-Bindung oder eine geradkettige oder verzweigte, gesättigte oder ungesättigte Alkylgruppe mit 1 bis 4 Kohlenstoffatomen mit der Maßgabe, daß, wenn $R_1$ Wasserstoff darstellt, A keine σ-Bindung bedeutet,
- $R_2$ und $R_3$ jeweils unabhängig voneinander
  - Wasserstoff,
  - eine geradkettige oder verzweigte und gegebenenfalls substituierte Alkylgruppe mit 1 bis 6 Kohlenstoffatomen,
  - eine Gruppe $-(CH_2)_nB$, worin n Werte von 0, 1, 2 und 3 annehmen kann und B ein gegebenenfalls substituiertes, gesättigtes, ungesättigtes oder aromatisches monocyclisches System mit 5 bis 7 Ringgliedern,

  oder $R_2$ und $R_3$ gemeinsam mit den Kohlenstoffatomen, an die sie gebunden sind, ein gesättigtes, gegebenenfalls substituiertes cyclisches System mit 5 bis 7 Ringgliedern,
- $R_4$ und $R_5$ jeweils unabhängig voneinander Wasserstoff, eine geradkettige oder verzweigte Alkylgruppe mit 1 bis 4 Kohlenstoffatomen oder eine Gruppe $-(CH_2)_nB$, wie sie bezüglich $R_2$ und $R_3$ definiert worden ist, bedeuten,

wobei der für die "mono- oder bicyclischen" Systeme verwendete Begriff "substituiert" bedeutet, daß diese Ringe durch eine oder mehrere (maximal 4) gleichartige oder verschiedene, gegebenenfalls verzweigte Niedrigalkylgruppen mit 1 bis 4 Kohlenstoffatomen, Nitrogruppen, Aminogruppen, gegebenenfalls verzweigte Niedrigalkoxygruppen mit 1 bis 4 Kohlenstoffatomen, Hydroxylgruppen, Trifluormethylgruppen, Halogenatome oder $SO_2R_6$-Gruppen substituiert sein können,
der mit dem Ausdruck Alkyl benutzte Begriff "substituiert" bedeutet, daß die Gruppe durch eine oder mehrere geradkettige oder verzweigte Niedrigalkoxygruppen mit 1 bis 4 Kohlenstoffatomen substituiert sein kann,

- worin $R_6$ eine gegebenenfalls verzweigte Niedrigalkylgruppe mit 1 bis 4 Kohlenstoffatomen, eine Phenylgruppe (die gegebenenfalls durch eine oder mehrere geradkettige oder verzweigte Niedrigalkyl- oder Niedrigalkoxygruppen mit 1 bis 4 Kohlenstoffatomen, Trifluormethylgruppen oder Halogenatome substituiert sein kann) oder eine Gruppe $-NR_7R_8$ darstellt, worin $R_7$ und $R_8$ unabhängig voneinander Wasserstoff oder eine gegebenenfalls verzweigte Niedrigalkylgruppe mit 1 bis 4 Kohlenstoffatomen darstellen,
- von deren Isomeren, Diastereoisomeren und Enantiomeren in isolierter Form oder in Form einer Mischung,
- und von deren Additionssalzen mit einer anorganischen oder organischen, pharmazeutisch annehmbaren Säure,

**dadurch gekennzeichnet**, daß man einen Aminoalkohol der allgemeinen Formel (II):

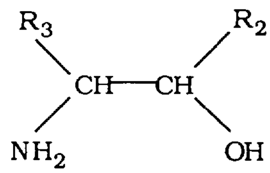

(II)

in der $R_2$ und $R_3$ die bezüglich der Verbindungen der allgemeinen Formel (I) angegebenen Bedeutungen besitzen, in wäßrigem Medium, in Gegenwart einer schwachen Säure und bei einer Temperatur zwischen 0 und 20°C mit Glutaraldehyd und dann mit oder ohne zwischenzeitliche Isolierung mit Kaliumcyanid bei einer Temperatur zwischen 10°C und 30°C umsetzt, so daß man nach der Isolierung und Reinigung mit Hilfe klassischer Verfahrensweisen, wie der Säulenchromatographie über Siliciumdioxid und/oder der Umkristallisation, eine bicyclische Verbindung der allgemeinen Formel (III) erhält:

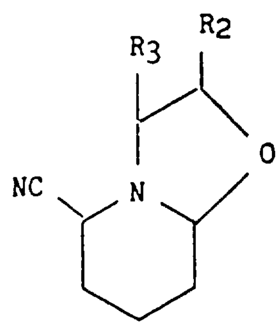

**(III)**

in der $R_2$ und $R_3$ die bezüglich der Verbindungen der allgemeinen Formel (I) angegebenen Bedeutungen besitzen, welche man gegebenenfalls in aprotischem Medium und bei einer Temperatur zwischen -78°C und Raumtemperatur mit einer lithiumorganischen Verbindung der allgemeinen Formel (IV) umsetzen kann:

$$R'_4Li \qquad (IV)$$

in der $R'_4$ die gleichen Bedeutungen besitzt wie $R_4$ bezüglich der Verbindungen der allgemeinen Formel (I), mit der Maßgabe, daß $R'_4$ kein Wasserstoffatom darstellt, so daß man ein Imin der allgemeinen Formel (V) erhält:

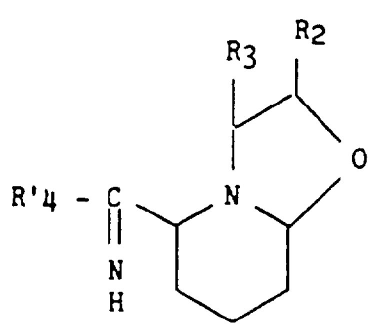

**(V)**

in der $R_2$ und $R_3$ die bezüglich der Verbindungen der allgemeinen Formel (I) angegebenen Bedeutungen besitzen und $R'_4$ die bezüglich der Verbindungen der allgemeinen Formel (IV) angegebenen Bedeutungen besitzt, welches man ebenso wie die Nitrile der allgemeinen Formel (III) mit einem Metallhydrid in aprotischem Medium umsetzen kann, so daß man nach der Isolierung und der eventuellen Reinigung mit Hilfe klassischer Verfahrensweisen, wie der Säulenchromatographie über Siliciumdioxid und/oder der Umkristallisation, eine Verbindung der allgemeinen Formel (VI) erhält:

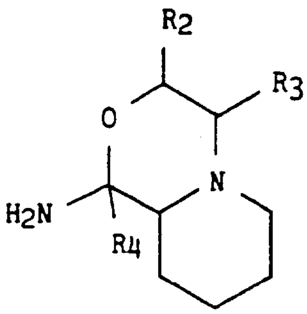

(VI)

in der $R_2$, $R_3$ und $R_4$ die bezüglich der Verbindungen der allgemeinen Formel (I) angegebenen Bedeutungen besitzen (wobei die Konfiguration der asymmetrischen Kohlenstoffatome der Verbindungen der allgemeinen Formel (III) und (V) die Konfiguration der asymmetrischen Kohlenstoffatome der Verbindungen der allgemeinen Formel (VI) bedingt), welche man:

- entweder mit einer Halogenverbindung der allgemeinen Formel (VII):

  $$R'_5\text{-}X \qquad \text{(VII)}$$

  in der $R'_5$ die Bedeutungen für $R_5$ bezüglich der Verbindungen der allgemeinen Formel (I) besitzt, mit der Maßgabe, daß $R'_5$ kein Wasserstoffatom darstellt, und X ein Halogenatom bedeutet, umsetzen kann, so daß man nach der Isolierung und der eventuellen Reinigung mit Hilfe klassischer Verfahrensweisen, wie der Säulenchromatographie über Siliciumdioxid und/oder der Umkristallisation, eine Verbindung der allgemeinen Formel (VIII) erhält:

  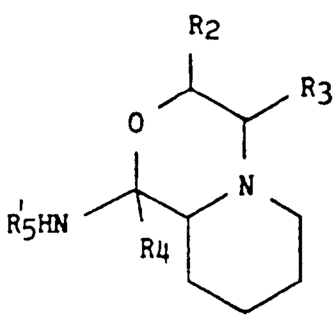

  (VIII)

  in der $R_2$, $R_3$ und $R_4$ die bezüglich der Verbindungen der allgemeinen Formel (I) angegebenen Bedeutungen besitzen, und $R'_5$ die bezüglich der Verbindungen der allgemeinen Formel (VII) angegebenen Bedeutungen besitzt, welche man anschließend gegebenenfalls in Gegenwart einer Base mit einem Säurechlorid der allgemeinen Formel (IX):

  $$R_1 - A - \underset{\underset{O}{\|}}{C}Cl \qquad \text{(IX)}$$

  in der $R_1$ und A die bezüglich der Verbindungen der allgemeinen Formel (I) angegebenen Bedeutungen besitzen, umsetzt, so daß man nach der Isolierung und der Reinigung mit Hilfe klassischer Verfahrensweisen, wie der Säulenchromatographie über Siliciumdioxid und/oder der Umkristallisation, die Verbindungen der allgemeinen Formel (I) erhält, in der $R_5$ kein Wasserstoffatom darstellt,
- oder direkt mit einem Säurechlorid der allgemeinen Formel (IX) umsetzt, so daß man nach der Isolierung und der Reinigung mit Hilfe klassischer Verfahrensweisen, wie der Säulenchromatographie über Siliciumdioxid und/ oder der Umkristallisation, die Verbindungen der allgemeinen Formel (I) erhält, in der $R_5$ ein Wasserstoffatom darstellt, welche man anschließend mit Hilfe klassischer Verfahrensweisen mit einer Verbindung der allgemeinen Formel (VII) N-alkylieren kann, so daß man nach der Isolierung und der eventuellen Reinigung mit Hilfe klassischer Verfahrensweisen, wie der Säulenchromatographie über Siliciumdioxid und/oder der Umkristallisation, die Verbindungen der allgemeinen Formel (I) erhält, worin $R_5$ kein Wasserstoffatom darstellt,
- mit der Maßgabe, daß die Verbindungen der allgemeinen Formel (I), wenn sie nicht Diastereoisomeren-rein sind, in ihre verschiedenen Diastereoisomere getrennt und/oder mit einer anorganischen oder organischen, pharmazeutisch annehmbaren Säure in ihre Salze überführt werden können.

2. Verfahren nach Anspruch 1 zur Herstellung von N-(4-Phenyl-octahydropyrido[2,1-c][1,4]oxazin-1-yl)-3,4,5-trimethoxybenzamid sowie von dessen Isomeren in isolierter Form oder in Form einer Mischung und von dessen Additionssalzen mit einer anorganischen oder organischen, pharmazeutisch annehmbaren Säure.

3. Verfahren nach Anspruch 1 zur Herstellung von N-(4-Phenyl-octahydropyrido[2,1-c][1,4]oxazin-1-yl)-3,4,5-trimethoxy-benzamid (1R) (4R) (9aS) sowie von dessen Additionssalzen mit einer anorganischen oder organischen, pharmazeutisch annehmbaren Säure:

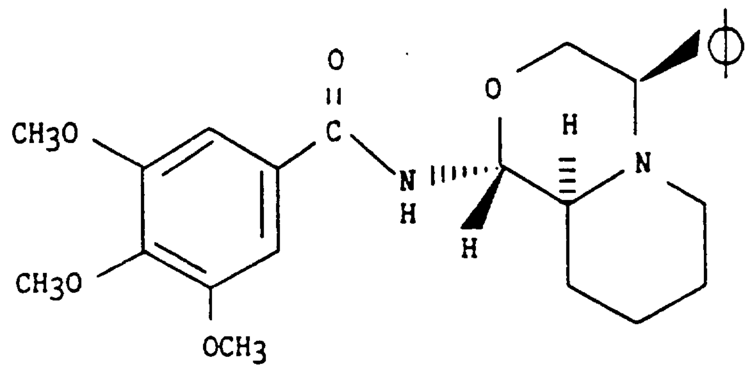

4. Verfahren nach Anspruch 1 zur Herstellung von N-(4-Phenyl-octahydropyrido-[2,1-c][1,4]oxazin-1-yl)-2,6-dimethoxy-3,5-dibrombenzamid sowie von dessen Isomeren in isolierter Form oder in Form einer Mischung und von dessen Additionssalzen mit einer anorganischen oder organischen, pharmazeutisch annehmbaren Säure.

5. Verfahren nach Anspruch 1 zur Herstellung von N-(1-Methyl-4-phenyl-octahydropyrido[2,1-c][1,4]oxazin-1-yl)-3,4,5-trimethoxybenzamid sowie von dessen Isomeren in isolierter Form oder in Form einer Mischung und von dessen Additionssalzen mit einer anorganischen oder organischen, pharmazeutisch annehmbaren Säure.

6. Verfahren nach Anspruch 1 zur Herstellung von N-(4-Methyl-3-phenyl-octahydropyrido[2,1-c][1,4]oxazin-1-yl)-3,4,5-trimethoxybenzamid sowie von dessen Isomeren in isolierter Form oder in Form einer Mischung und von dessen Additionssalzen mit einer anorganischen oder organischen, pharmazeutisch annehmbaren Säure.

7. Verfahren zur Herstellung der Verbindungen der allgemeinen Formel (VI):

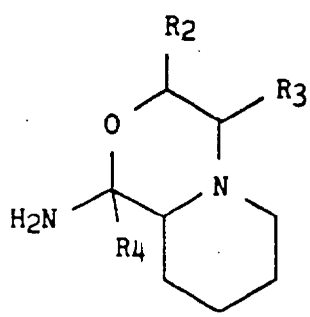

(VI)

in der $R_2$, $R_3$ und $R_4$ die bezüglich der Formel (I) in Anspruch 1 angegebenen Bedeutungen besitzen, sowie von deren Isomeren in isolierter Form oder in Form einer Mischung und von deren Additionssalzen mit einer anorganischen oder organischen Säure, die als Synthesezwischenprodukte für die Herstellung der Verbindungen der allgemeinen Formel (I) geeignet sind, **dadurch gekennzeichnet**, daß man
einen Aminoalkohol der allgemeinen Formel (II):

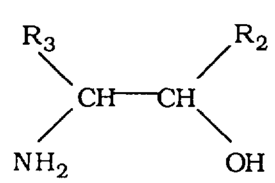

(II)

in der $R_2$ und $R_3$ die bezüglich der allgemeinen Formel (I) angegebenen Bedeutungen besitzen, in wäßrigem

Medium, in Gegenwart einer schwachen Säure und bei einer Temperatur zwischen 0 und 20°C mit Glutaraldehyd umsetzt und dann mit oder ohne zwischenzeitliche Isolierung mit Kaliumcyanid bei einer Temperatur zwischen 10°C und 30°C umsetzt, so daß man nach der Isolierung und Reinigung mit Hilfe klassischer Verfahrensweisen, wie der Säulenchromatographie über Siliciumdioxid und/oder der Umkristallisation, eine bicyclische Verbindung der allgemeinen Formel (III) erhält:

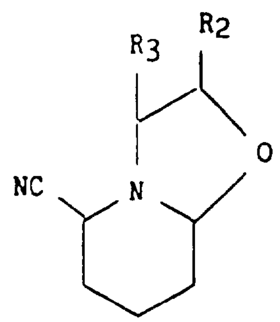

(III)

in der $R_2$ und $R_3$ die bezüglich der Verbindungen der allgemeinen Formel (I) angegebenen Bedeutungen besitzen, die man gegebenenfalls in aprotischem Medium und bei einer Temperatur zwischen -78°C und Raumtemperatur mit einer lithiumorganischen Verbindung der allgemeinen Formel (IV):

$$R'_4Li \qquad (IV)$$

in der $R'_4$ die gleichen Bedeutungen besitzt wie $R_4$ für die Verbindungen der allgemeinen Formel (I), mit der Maßgabe, daß $R'_4$ kein Wasserstoffatom darstellt, umsetzen kann, so daß man ein Imin der allgemeinen Formel (V) erhält:

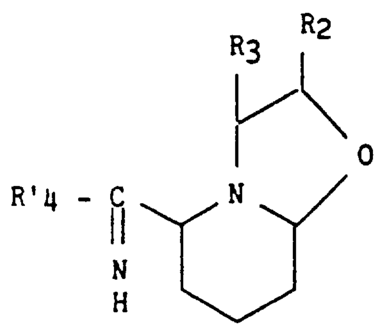

(V)

in der $R_2$ und $R_3$ die für die Verbindungen der allgemeinen Formel (I) angegebenen Bedeutungen besitzen und $R'_4$ die für die Verbindungen der allgemeinen Formel (IV) angegebenen Bedeutungen besitzt, welches man ebenso wie die Nitrile der allgemeinen Formel (III) mit einem Metallhydrid in aprotischem Medium umsetzen kann, so daß man nach der Isolierng und der eventuellen Reinigung mit Hilfe klassicher Verfahrensweisen, wie der Säulenchromatographie über Siliciumdioxid und/oder der Umkristallisation, die Verbindungen der allgemeinen Formel (VI) erhält:

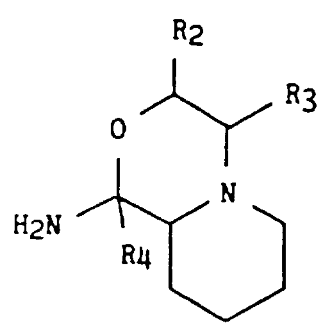

(VI)

in der $R_2$, $R_3$ und $R_4$ die bezüglich der Verbindungen der allgemeinen Formel (I) angegebenen Bedeutungen besitzen (wobei die Konfiguration der asymmetrischen Kohlenstoffatome der Verbindungen der allgemeinen Formel (III) und (V) die Konfiguration der asymmetrischen Kohlenstoffatome der Verbindungen der allgemeinen Formel (VI) bedingt).

8. Verfahren zur Herstellung einer pharmazeutischen Zubereitung, die als Wirkstoff eine Verbindung hergestellt nach einem der Ansprüche bis 6 allein oder in Kombination mit einem oder mehreren inerten, nichttoxischen, pharmazeutisch annehmbaren Trägermaterialien oder Bindemitteln enthält.

9. Verfahren zur Herstellung einer pharmazeutischen Zubereitung nach Anspruch 8 in einer Form, die insbesondere zur Behandlung von Psycho-Verhaltensstörungen geeignet ist.

**Patentansprüche für folgenden Vertragsstaat : GR**

1. Verfahren zur Herstellung der 1 -Acylamino-octyhydropyrido[2,1-c][1,4]-oxazine der allgemeinen Formel (I):

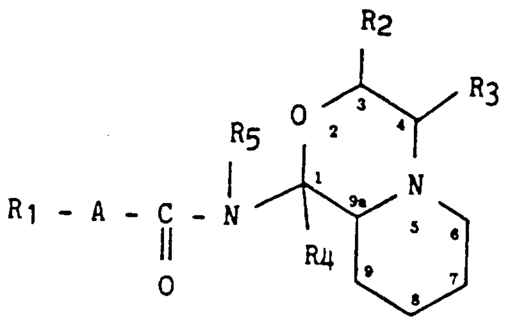

(I)

in der:

- $R_1$ Wasserstoff oder ein gegebenenfalls substituiertes mono- oder bicyclisches System ausgewählt aus Phenyl, Naphthyl, Pyridyl, Thienyl, Furyl, Chinolyl, Isochinolyl, Indolyl, Indolinyl, Perhydroindolyl und Benzofuryl,
- A eine σ-Bindung oder eine geradkettige oder verzweigte, gesättigte oder ungesättigte Alkylgruppe mit 1 bis 4 Kohlenstoffatomen mit der Maßgabe, daß, wenn $R_1$ Wasserstoff darstellt, A keine σ-Bindung bedeutet,
- $R_2$ und $R_3$ jeweils unabhängig voneinander
  - Wasserstoff,
  - eine geradkettige oder verzweigte und gegebenenfalls substituierte Alkylgruppe mit 1 bis 6 Kohlenstoffatomen,
  - eine Gruppe $-(CH_2)_nB$, worin n Werte von 0, 1, 2 und 3 annehmen kann und B ein gegebenenfalls substituiertes, gesättigtes, ungesättigtes oder aromatisches monocyclisches System mit 5 bis 7 Ringgliedern,

  oder $R_2$ und $R_3$ gemeinsam mit den Kohlenstoffatomen, an die sie gebunden sind, ein gesättigtes, gegebenenfalls substituiertes cyclisches System mit 5 bis 7 Ringgliedern,
- $R_4$ und $R_5$ jeweils unabhängig voneinander Wasserstoff, eine geradkettige oder verzweigte Alkylgruppe mit 1 bis 4 Kohlenstoffatomen oder eine Gruppe $-(CH_2)_nB$, wie sie bezüglich $R_2$ und $R_3$ definiert worden ist, bedeuten,

wobei der für die "mono- oder bicyclischen" Systeme verwendete Begriff "substituiert" bedeutet, daß diese Ringe durch eine oder mehrere (maximal 4) gleichartige oder verschiedene, gegebenenfalls verzweigte Niedrigalkylgruppen mit 1 bis 4 Kohlenstoffatomen, Nitrogruppen, Aminogruppen, gegebenenfalls verzweigte Niedrigalkoxygruppen mit 1 bis 4 Kohlenstoffatomen, Hydroxylgruppen, Trifluormethylgruppen, Halogenatome oder $SO_2R_6$-Gruppen substituiert sein können,
der mit dem Ausdruck Alkyl benutzte Begriff "substituiert" bedeutet, daß die Gruppe durch eine oder mehrere geradkettige oder verzweigte Niedrigalkoxygruppen mit 1 bis 4 Kohlenstoffatomen substituiert sein kann,

- worin $R_6$ eine gegebenenfalls verzweigte Niedrigalkylgruppe mit 1 bis 4 Kohlenstoffatomen, eine Phenylgruppe (die gegebenenfalls durch eine oder mehrere geradkettige oder verzweigte Niedrigalkyl- oder Niedrigalkoxygruppen mit 1 bis 4 Kohlenstoffatomen, Trifluormethylgruppen oder Halogenatome substituiert sein kann) oder eine Gruppe $-NR_7R_8$ darstellt, worin $R_7$ und $R_8$ unabhängig voneinander Wasserstoff oder eine gegebenenfalls verzweigte Niedrigalkylgruppe mit 1 bis 4 Kohlenstoffatomen darstellen,

- von deren Isomeren, Diastereoisomeren und Enantiomeren in isolierter Form oder in Form einer Mischung,
- und von deren Additionssalzen mit einer anorganischen oder organischen, pharmazeutisch annehmbaren Säure,

**dadurch gekennzeichnet**, daß man einen Aminoalkohol der allgemeinen Formel (II):

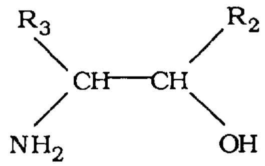

(II)

in der $R_2$ und $R_3$ die bezüglich der Verbindungen der allgemeinen Formel (I) angegebenen Bedeutungen besitzen, in wäßrigem Medium, in Gegenwart einer schwachen Säure und bei einer Temperatur zwischen 0 und 20°C mit Glutaraldehyd und dann mit oder ohne zwischenzeitliche Isolierung mit Kaliumcyanid bei einer Temperatur zwischen 10°C und 30°C umsetzt, so daß man nach der Isolierung und Reinigung mit Hilfe klassischer Verfahrensweisen, wie der Säulenchromatographie über Siliciumdioxid und/oder der Umkristallisation, eine bicyclische Verbindung der allgemeinen Formel (III) erhält:

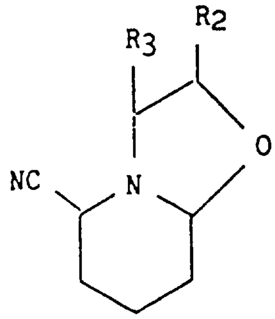

(III)

in der $R_2$ und $R_3$ die bezüglich der Verbindungen der allgemeinen Formel (I) angegebenen Bedeutungen besitzen, welche man gegebenenfalls in aprotischem Medium und bei einer Temperatur zwischen -78°C und Raumtemperatur mit einer lithiumorganischen Verbindung der allgemeinen Formel (IV) umsetzen kann:

$$R'_4Li \qquad (IV)$$

in der $R'_4$ die gleichen Bedeutungen besitzt wie $R_4$ bezüglich der Verbindungen der allgemeinen Formel (I), mit der Maßgabe, daß $R'_4$ kein Wasserstoffatom darstellt, so daß man ein Imin der allgemeinen Formel (V) erhält:

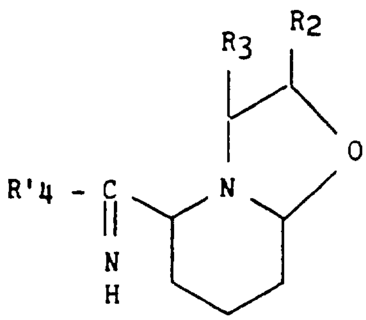

(V)

in der $R_2$ und $R_3$ die bezüglich der Verbindungen der allgemeinen Formel (I) angegebenen Bedeutungen besitzen und $R'_4$ die bezüglich der Verbindungen der allgemeinen Formel (IV) angegebenen Bedeutungen besitzt, welches man ebenso wie die Nitrile der allgemeinen Formel (III) mit einem Metallhydrid in aprotischem Medium umsetzen kann, so daß man nach der Isolierung und der eventuellen Reinigung mit Hilfe klassischer Verfahrensweisen, wie der Säulenchromatographie über Siliciumdioxid und/oder der Umkristallisation, eine Verbindung der allgemeinen Formel (VI) erhält:

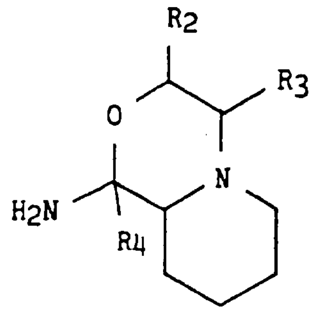

(VI)

in der $R_2$, $R_3$ und $R_4$ die bezüglich der Verbindungen der allgemeinen Formel (I) angegebenen Bedeutungen besitzen (wobei die Konfiguration der asymmetrischen Kohlenstoffatome der Verbindungen der allgemeinen Formel (III) und (V) die Konfiguration der asymmetrischen Kohlenstoffatome der Verbindungen der allgemeinen Formel (VI) bedingt), welche man:

- entweder mit einer Halogenverbindung der allgemeinen Formel (VII):

  $$R'_5\text{-}X \qquad (VII)$$

  in der $R'_5$ die Bedeutungen für $R_5$ bezüglich der Verbindungen der allgemeinen Formel (I) besitzt, mit der Maßgabe, daß $R'_5$ kein Wasserstoffatom darstellt, und X ein Halogenatom bedeutet, umsetzen kann, so daß man nach der Isolierung und der eventuellen Reinigung mit Hilfe klassischer Verfahrensweisen, wie der Säulenchromatographie über Siliciumdioxid und/oder der Umkristallisation, eine Verbindung der allgemeinen Formel (VIII) erhält:

  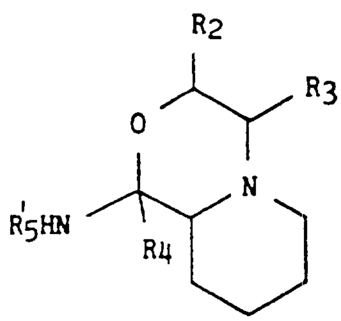

  (VIII)

  in der $R_2$, $R_3$ und $R_4$ die bezüglich der Verbindungen der allgemeinen Formel (I) angegebenen Bedeutungen besitzen, und $R'_5$ die bezüglich der Verbindungen der allgemeinen Formel (VII) angegebenen Bedeutungen besitzt, welche man anschließend gegebenenfalls in Gegenwart einer Base mit einem Säurechlorid der allgemeinen Formel (IX):

  $$R_1-A-\underset{\underset{O}{\|}}{C}Cl \qquad (IX)$$

  in der $R_1$ und A die bezüglich der Verbindungen der allgemeinen Formel (I) angegebenen Bedeutungen besitzen, umsetzt, so daß man nach der Isolierung und der Reinigung mit Hilfe klassischer Verfahrensweisen, wie der Säulenchromatographie über Siliciumdioxid und/oder der Umkristallisation, die Verbindungen der allgemeinen Formel (I) erhält, in der $R_5$ kein Wasserstoffatom darstellt,
- oder direkt mit einem Säurechlorid der allgemeinen Formel (IX) umsetzt, so daß man nach der Isolierung und der Reinigung mit Hilfe klassischer Verfahrensweisen, wie der Säulenchromatographie über Siliciumdioxid und/ oder der Umkristallisation, die Verbindungen der allgemeinen Formel (I) erhält, in der $R_5$ ein Wasserstoffatom darstellt, welche man anschließend mit Hilfe klassischer Verfahrensweisen mit einer Verbindung der allgemeinen Formel (VII) N-alkylieren kann, so daß man nach der Isolierung und der eventuellen Reinigung mit Hilfe klassischer Verfahrensweisen, wie der Säulenchromatographie über Siliciumdioxid und/oder der Umkristallisation, die Verbindungen der allgemeinen Formel (I) erhält, worin $R_5$ kein Wasserstoffatom darstellt,
- mit der Maßgabe, daß die Verbindungen der allgemeinen Formel (I), wenn sie nicht Diastereoisomeren-rein sind, in ihre verschiedenen Diastereoisomere getrennt und/oder mit einer anorganischen oder organischen,

pharmazeutisch annehmbaren Säure in ihre Salze überführt werden können.

**2.** Verfahren nach Anspruch 1 zur Herstellung von N-(4-Phenyl-octahydropyrido[2,1-c][1,4]oxazin-1-yl)-3,4,5-trimethoxybenzamid sowie von dessen Isomeren in isolierter Form oder in Form einer Mischung und von dessen Additionssalzen mit einer anorganischen oder organischen, pharmazeutisch annehmbaren Säure.

**3.** Verfahren nach Anspruch 1 zur Herstellung von N-(4-Phenyl-octahydropyrido[2,1-c][1,4]oxazin-1-yl)-3,4,5-trimethoxy-benzamid (1R) (4R) (9aS) sowie von dessen Additionssalzen mit einer anorganischen oder organischen, pharmazeutisch annehmbaren Säure:

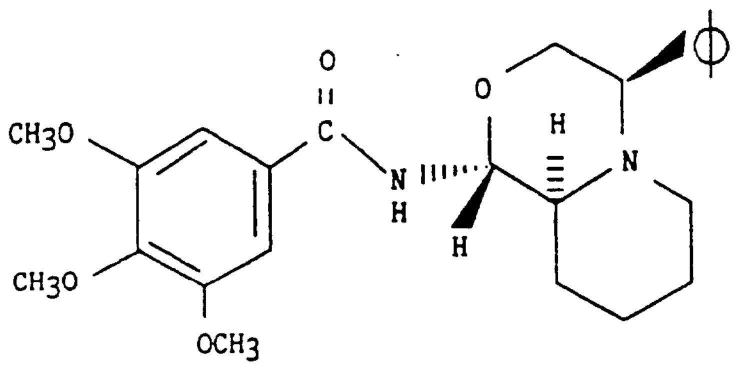

**4.** Verfahren nach Anspruch 1 zur Herstellung von N-(4-Phenyl-octahydropyrido-[2,1-c][1,4]oxazin-1-yl)-2,6-dimethoxy-3,5-dibrombenzamid sowie von dessen Isomeren in isolierter Form oder in Form einer Mischung und von dessen Additionssalzen mit einer anorganischen oder organischen, pharmazeutisch annehmbaren Säure.

**5.** Verfahren nach Anspruch 1 zur Herstellung von N-(1-Methyl-4-phenyl-octahydropyrido[2,1-c][1,4]oxazin-1-yl)-3,4,5-trimethoxybenzamid sowie von dessen Isomeren in isolierter Form oder in Form einer Mischung und von dessen Additionssalzen mit einer anorganischen oder organischen, pharmazeutisch annehmbaren Säure.

**6.** Verfahren nach Anspruch 1 zur Herstellung von N-(4-Methyl-3-phenyl-octahydropyrido[2,1-c][1,4]oxazin-1-yl)-3,4,5-trimethoxybenzamid sowie von dessen Isomeren in isolierter Form oder in Form einer Mischung und von dessen Additionssalzen mit einer anorganischen oder organischen, pharmazeutisch annehmbaren Säure.

**7.** Verfahren zur Herstellung der Verbindungen der allgemeinen Formel (VI):

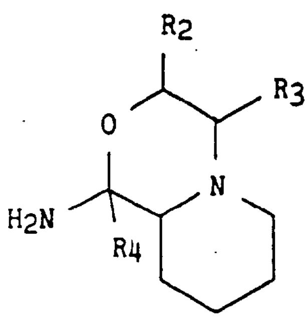

(VI)

in der $R_2$, $R_3$ und $R_4$ die bezüglich der Formel (I) in Anspruch 1 angegebenen Bedeutungen besitzen, sowie von deren Isomeren in isolierter Form oder in Form einer Mischung und von deren Additionssalzen mit einer anorganischen oder organischen Säure, die als Synthesezwischenprodukte für die Herstellung der Verbindungen der allgemeinen Formel (I) geeignet sind, **dadurch gekennzeichnet**, daß man
einen Aminoalkohol der allgemeinen Formel (II):

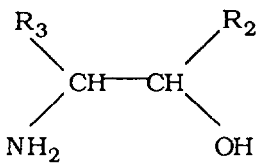

(II)

in der $R_2$ und $R_3$ die bezüglich der allgemeinen Formel (I) angegebenen Bedeutungen besitzen, in wäßrigem Medium, in Gegenwart einer schwachen Säure und bei einer Temperatur zwischen 0 und 20°C mit Glutaraldehyd umsetzt und dann mit oder ohne zwischenzeitliche Isolierung mit Kaliumcyanid bei einer Temperatur zwischen 10°C und 30°C umsetzt, so daß man nach der Isolierung und Reinigung mit Hilfe klassischer Verfahrensweisen, wie der Säulenchromatographie über Siliciumdioxid und/oder der Umkristallisation, eine bicyclische Verbindung der allgemeinen Formel (III) erhält:

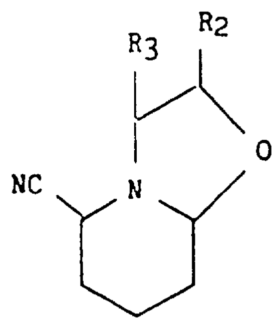

(III)

in der $R_2$ und $R_3$ die bezüglich der Verbindungen der allgemeinen Formel (I) angegebenen Bedeutungen besitzen, die man gegebenenfalls in aprotischem Medium und bei einer Temperatur zwischen -78°C und Raumtemperatur mit einer lithiumorganischen Verbindung der allgemeinen Formel (IV):

$$R'_4Li \qquad (IV)$$

in der $R'_4$ die gleichen Bedeutungen besitzt wie $R_4$ für die Verbindungen der allgemeinen Formel (I), mit der Maßgabe, daß $R'_4$ kein Wasserstoffatom darstellt, umsetzen kann, so daß man ein Imin der allgemeinen Formel (V) erhält:

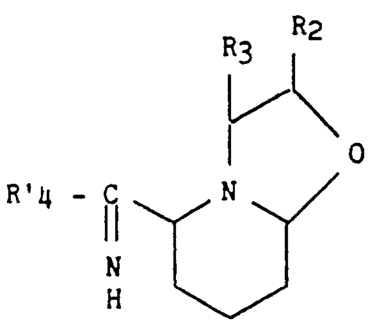

(V)

in der $R_2$ und $R_3$ die für die Verbindungen der allgemeinen Formel (I) angegebenen Bedeutungen besitzen und $R'_4$ die für die Verbindungen der allgemeinen Formel (IV) angegebenen Bedeutungen besitzt, welches man ebenso wie die Nitrile der allgemeinen Formel (III) mit einem Metallhydrid in aprotischem Medium umsetzen kann, so daß man nach der Isolierng und der eventuellen Reinigung mit Hilfe klassicher Verfahrensweisen, wie der Säulenchromatographie über Siliciumdioxid und/oder der Umkristallisation, die Verbindungen der allgemeinen Formel (VI) erhält:

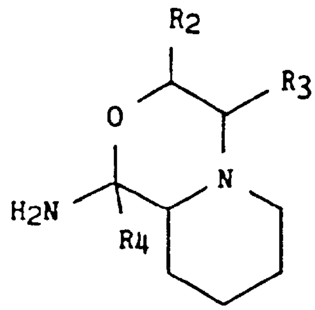

(VI)

in der $R_2$, $R_3$ und $R_4$ die bezüglich der Verbindungen der allgemeinen Formel (I) angegebenen Bedeutungen besitzen (wobei die Konfiguration der asymmetrischen Kohlenstoffatome der Verbindungen der allgemeinen Formel (III) und (V) die Konfiguration der asymmetrischen Kohlenstoffatome der Verbindungen der allgemeinen Formel (VI) bedingt).

**8.** Verfahren zur Herstellung einer pharmazeutischen Zubereitung, die als Wirkstoff eine Verbindung hergestellt nach einem der Ansprüche bis 6 allein oder in Kombination mit einem oder mehreren inerten, nichttoxischen, pharmazeutisch annehmbaren Trägermaterialien oder Bindemitteln enthält.

**9.** Verfahren zur Herstellung einer pharmazeutischen Zubereitung nach Anspruch 8 in einer Form, die insbesondere zur Behandlung von Psycho-Verhaltensstörungen geeignet ist.

## Claims

**Claims for the following Contracting States : AT, BE, CH, DE, DK, FR, GB, IT, LI, LU, MC, NL, PT, SE**

**1.** 1-Acylaminooctahydropyrido[2,1-c][1,4]oxazines of the general formula (I):

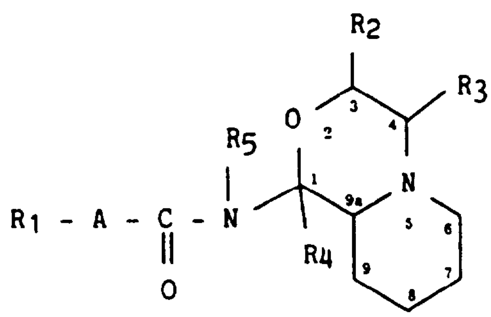

(I)

wherein :

- $R_1$ represents a hydrogen atom or a mono- or bicyclic system selected from phenyl, naphthyl, pyridyl, thienyl, furyl, quinolyl, isoquinolyl, indolyl, indolinyl, perhydroindolyl and benzofuryl, that is optionally substituted,
- A represents a σ bond or a straight-chained or branched, saturated or unsaturated, alkyl group having from 1 to 4 carbon atoms, with the proviso that when $R_1$ represents a hydrogen atom A cannot represent a σ bond,
- $R_2$ and $R_3$ each represents, independently of the other,
  - a hydrogen atom,
  - an optionally substituted, straight-chained or branched alkyl group having from 1 to 6 carbon atoms,
  - a $-(CH_2)_nB$ group in which n may be 0, 1, 2 or 3 and B represents a substituted or unsubstituted, saturated, unsaturated or aromatic monocyclic system having from 5 to 7 ring members,

  or $R_2$ and $R_3$ form together with the carbon atoms to which they are bonded an optionally substituted saturated cyclic system having from 5 to 7 ring members,

- $R_4$ and $R_5$ each represents, independently of the other, a hydrogen atom, a straight-chain or branched alkyl group having from 1 to 4 carbon atoms or a $-(CH_2)_nB$ group as defined in the description of $R_2$ and $R_3$,

the term "substituted" associated with "mono- or bicyclic" systems denotes that the rings may be substituted by one or more (4 maximum) identical or different groups selected from branched and unbranched lower alkyl having from 1 to 4 carbons atoms, nitro, amino, branched and unbranched lower alkoxy having from 1 to 4 carbon atoms, hydroxy, trifluoromethyl, halogen and $SO_2R_6$,
the term "substituted" associated with the term "alkyl" denotes that that group may be substituted by one or more straight-chained or branched lower alkoxy groups having from 1 to 4 carbon atoms,

- $R_6$ represents a branched or unbranched lower alkyl group having from 1 to 4 carbon atoms, a phenyl group (optionally substituted by one or more groups straight-chained or branched lower alkyl or alkoxy having from 1 to 4 carbon atoms, trifluoromethyl or halogen) or a $-NR_7R_8$ group in which $R_7$ and $R_8$ may each represent, independently of the other, a hydrogen atom or a branched or unbranched lower alkyl group having from 1 to 4 carbon atoms,

- their isomers, diastereoisomers and enantiomers, isolated or in the form of a mixture,

- their addition salts with a pharmaceutically acceptable mineral or organic acid.

**2.** Compound according to claim 1), which is N-(4-phenyloctahydropyrido[2,1-c] [1,4]oxazin-1-yl)-3,4,5-trimethoxybenzamide and also its isomers, isolated or in the form of a mixture, and their addition salts with a pharmaceutically acceptable mineral or organic acid.

**3.** Compound according to claims 1) and 2), which is (1R), (4R), (9aS) -N-(4-phenyloctahydropyrido[2,1-c] [1,4]oxazin-1-yl)-3,4,5-trimethoxybenzamide and also its addition salts with a pharmaceutically acceptable mineral or organic acid

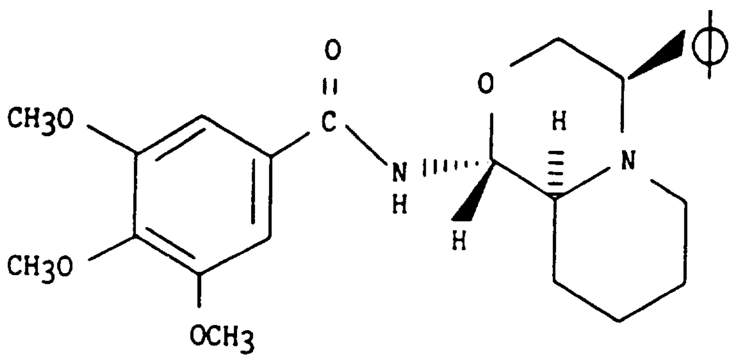

**4.** Compound according to claim 1), which is N-(4-phenyloctahydropyrido [2,1-c] [1,4] oxazin-1-yl)-2,6-dimethoxy-3,5-dibromobenzamide and also its isomers, isolated or in the form of a mixture, and their addition salts with a pharmaceutically acceptable mineral or organic acid.

**5.** Compound according to claim 1), which is N-(1-methyl-4-phenyloctahydropyrido [2,1-c] [1,4] oxazin-1-yl)-3,4,5-trimethoxybenzamide and also its isomers, isolated or in the form of a mixture, and their addition salts with a pharmaceutically acceptable mineral or organic acid.

**6.** Compound according to claim 1), which is N-(4-methyl-3-phenyloctahydropyrido [2,1-c] [1,4]oxazin-1-yl)-3,4,5-trimethoxybenzamide and also its isomers, isolated or in the form of a mixture, and their addition salts with a pharmaceutically acceptable mineral or organic acid.

**7.** Process for obtaining compounds of the general formula (I) according to claim 1), characterised in that an amino alcohol of the general formula (II) :

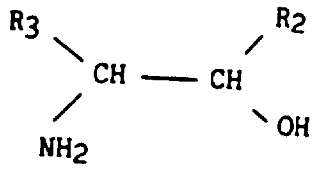

(II),

wherein $R_2$ and $R_3$ are as defined for compounds of the general formula (I), is reacted in aqueous medium, in the presence of a weak acid and at a temperature of from 0 to 20°C, with glutaraldehyde and then, with or without intermediate isolation, with potassium cyanide at a temperature of from 10°C to 30°C to obtain, after isolation and purification by conventional methods, such as chromatography on a silica column and/or recrystallisation, a bicyclic compound of the general formula (III) :

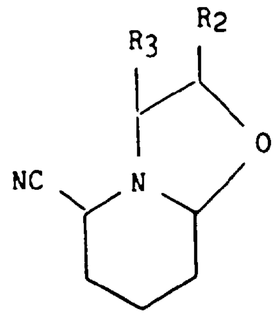

(III)

wherein $R_2$ and $R_3$ are as defined for compounds of the general formula (I), which may optionally be reacted, in an aprotic medium and at a temperature of from -78°C to room temperature, with an organolithium compound of the general formula (IV) :

$$R'_4Li \qquad (IV)$$

wherein $R'_4$ has the same meaning as $R_4$ in the compounds of the general formula (I), with the proviso that $R'_4$ cannot represent a hydrogen atom, to obtain an imine of the general formula (V) :

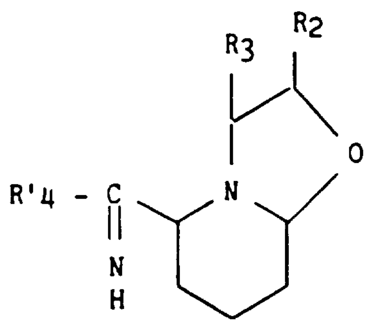

(V)

wherein $R_2$ and $R_3$ are as defined for compounds of the general formula (I) and $R'_4$ is as defined for compounds of the general formula (IV), which compound of formula (V) may be reacted, in the same manner as the nitriles of the general formula (III), with a metallic hydride in aprotic medium to obtain, after isolation and where necessary purification by conventional methods, such as chromatography on a silica column and/or recrystallisation, a compound of the general formula (VI) :

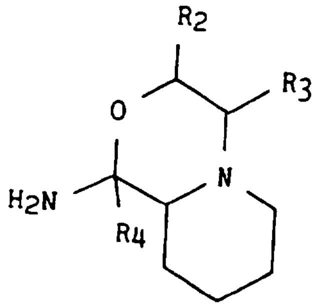

(VI)

wherein $R_2$, $R_3$, $R_4$ are as defined for compounds of the general formula (I) (the configuration of the asymmetric carbon atoms of the compounds of the general formulae (III) and (V) being carried over to the asymmetric carbon atoms of the compounds of the general formula (VI)), which may be reacted :

- either with a halogenated compound of the general formula (VII) :

  $$R'_5\text{-}X \qquad \text{(VII)}$$

  wherein $R'_5$ has the same meaning as $R_5$ in the compounds of the general formula (I), with the proviso that $R'_5$ cannot represent a hydrogen atom, and X represents a halogen atom, to obtain, after isolation and where necessary purification by conventional methods, such as chromatography on a silica column and/or recrystallisation, a compound of the general formula (VIII) :

  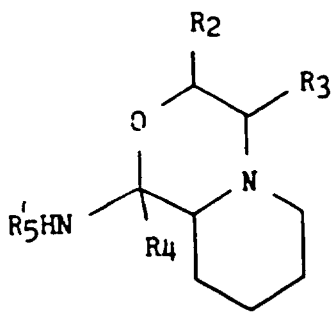

  (VIII)

  wherein $R_2$, $R_3$ and $R_4$ are as defined for compounds of the general formula (I) and $R'_5$ is as defined for the compounds of the general formula (VII), which is then reacted, optionally in the presence of a base, with an acid chloride of the general formula (IX) :

  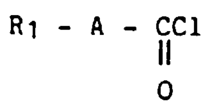

  (IX)

  wherein $R_1$ and A are as defined for compounds of the general formula (I) to obtain, after isolation and purification by conventional methods, such as chromatography on a silica column and/or recrystallisation, compounds of the general formula (I) wherein $R_5$ does not represent a hydrogen atom,

- or directly with an acid chloride of the general formula (IX) to obtain, after isolation and purification by conventional methods, such as chromatography on a silica column and/or recrystallisation, compounds of the general formula (I) wherein $R_5$ is a hydrogen atom, which may then be N-alkylated according to conventional methods by a compound of the general formula (VII) to obtain, after isolation and where necessary purification by conventional methods, such as chromatography on a silica column and/or recrystallisation, compounds of the general formula (I) wherein $R_5$ does not represent a hydrogen atom,

- it being understood that when the compounds of the general formula (I) are not in the form of pure diastereoisomers they may be separated into their different diastereoisomers and/or be converted into salts with a pharmaceutically acceptable mineral or organic acid.

**8.** Compounds of the general formula (VI) :

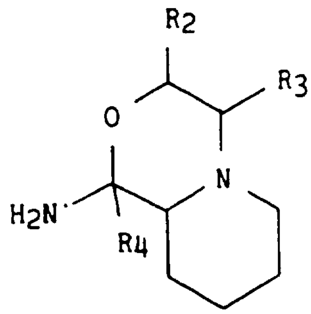

(VI)

wherein $R_2$, $R_3$ and $R_4$ are as defined in claim 1), as well as their isomers, isolated or in the form of a mixture, and their addition salts with a mineral or organic acid, which can be used as synthesis intermediates for the preparation of compounds of the general formula (I) according to claim 1).

**9.** Pharmaceutical compositions comprising as active ingredient a compound according to claims 1) to 6), alone or in combination with one or more pharmaceutically acceptable inert non-toxic excipients or carriers.

**10.** Pharmaceutical compositions according to claim 9) presented in a form suitable especially for the treatment of psycho-behavioural disorders.

**Claims for the following Contracting State : ES**

**1.** Process for the preparation of 1-acylaminooctahydropyrido [2,1-c] [1,4]oxazines of the general formula (I) :

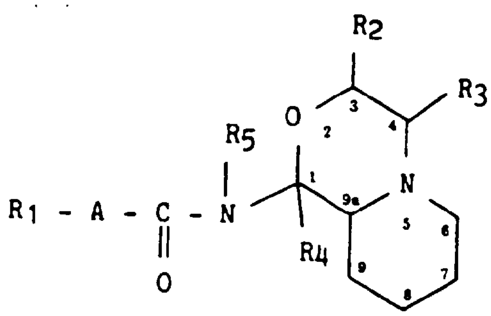

(I)

wherein :

- $R_1$ represents a hydrogen atom or a mono- or bicyclic system selected from phenyl, naphthyl, pyridyl, thienyl, furyl, quinolyl, isoquinolyl, indolyl, indolinyl, perhydroindolyl and benzofuryl, that is optionally substituted,
- A represents a σ bond or a straight-chained or branched, saturated or unsaturated, alkyl group having from 1 to 4 carbon atoms, with the proviso that when $R_1$ represents a hydrogen atom A cannot represent a σ bond,
- $R_2$ and $R_3$ each represents, independently of the other,
  - a hydrogen atom,
  - an optionally substituted, straight-chained or branched alkyl group having from 1 to 6 carbon atoms,
  - a $-(CH_2)_nB$ group in which n may be 0, 1, 2 or 3 and B represents a substituted or unsubstituted, saturated, unsaturated or aromatic monocyclic system having from 5 to 7 ring members,

  or $R_2$ and $R_3$ form together with the carbon atoms to which they are bonded an optionally substituted saturated cyclic system having from 5 to 7 ring members,
- $R_4$ and $R_5$ each represents, independently of the other, a hydrogen atom, a straight-chain or branched alkyl group having from 1 to 4 carbon atoms or a $-(CH_2)_nB$ group as defined in the description of $R_2$ and $R_3$,

the term "substituted" associated with "mono- or bicyclic" systems denotes that the rings may be substituted by one or more (4 maximum) identical or different groups selected from branched and unbranched lower alkyl having from 1 to 4 carbons atoms, nitro, amino, branched and unbranched lower alkoxy having from 1 to 4 carbon atoms, hydroxy, trifluoromethyl, halogen and $SO_2R_6$,
the term "substituted" associated with the term "alkyl" denotes that that group may be substituted by one or more straight-chained or branched lower alkoxy groups having from 1 to 4 carbon atoms,

- $R_6$ represents a branched or unbranched lower alkyl group having from 1 to 4 carbon atoms, a phenyl group (optionally substituted by one or more groups straight-chained or branched lower alkyl or alkoxy having from 1 to 4 carbon atoms, trifluoromethyl or halogen) or a $-NR_7R_8$ group in which $R_7$ and $R_8$ may each represent, independently of the other, a hydrogen atom or a branched or unbranched lower alkyl group having from 1 to 4 carbon atoms,

- their isomers, diastereoisomers and enantiomers, isolated or in the form of a mixture,

- and their addition salts with a pharmaceutically acceptable mineral or organic acid,

characterised in that :
an amino alcohol of the general formula (II) :

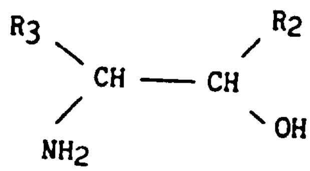

(II),

wherein $R_2$ and $R_3$ are as defined for compounds of the general formula (I), is reacted in aqueous medium, in the presence of a weak acid and at a temperature of from 0 to 20°C, with glutaraldehyde and then, with or without intermediate isolation, with potassium cyanide at a temperature of from 10°C to 30°C to obtain, after isolation and purification by conventional methods, such as chromatography on a silica column and/or recrystallisation, a bicyclic compound of the general formula (III) :

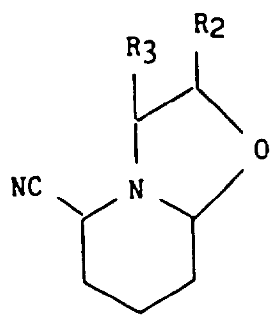

(III)

wherein $R_2$ and $R_3$ are as defined for compounds of the general formula (I), which may optionally be reacted, in an aprotic medium and at a temperature of from -78°C to room temperature, with an organolithium compound of the general formula (IV) :

$$R'_4Li \quad (IV)$$

wherein $R'_4$ has the same meaning as $R_4$ in the compounds of the general formula (I), with the proviso that $R'_4$ cannot represent a hydrogen atom, to obtain an imine of the general formula (V) :

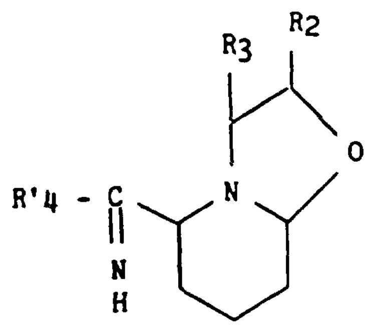

(V)

wherein $R_2$ and $R_3$ are as defined for compounds of the general formula (I) and $R'_4$ is as defined for compounds of the general formula (IV), which compound of formula (V) may be reacted, in the same manner as the nitriles of the general formula (III), with a metallic hydride in aprotic medium to obtain, after isolation and where necessary purification by conventional methods, such as chromatography on a silica column and/or recrystallisation, a compound of the general formula (VI) :

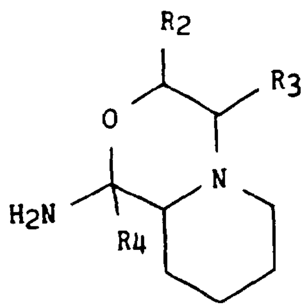

(VI)

wherein $R_2$, $R_3$, $R_4$ are as defined for compounds of the general formula (I) (the configuration of the asymmetric carbon atoms of the compounds of the general formulae (III) and (V) being carried over to the asymmetric carbon atoms of the compounds of the general formula (VI)), which may be reacted :

- either with a halogenated compound of the general formula (VII) :

$$R'_5\text{-}X \qquad (VII)$$

  wherein $R'_5$ has the same meaning as $R_5$ in the compounds of the general formula (I), with the proviso that $R'_5$ cannot represent a hydrogen atom, and X represents a halogen atom, to obtain, after isolation and where necessary purification by conventional methods, such as chromatography on a silica column and/or recrystallisation, a compound of the general formula (VIII) :

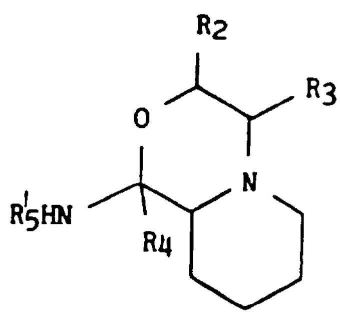

(VIII)

  wherein $R_2$, $R_3$ and $R_4$ are as defined for compounds of the general formula (I) and $R'_5$ is as defined for the compounds of the general formula (VII), which is then reacted, optionally in the presence of a base, with an acid chloride of the general formula (IX) :

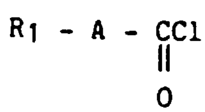

(IX)

wherein $R_1$ and A are as defined for compounds of the general formula (I) to obtain, after isolation and purification by conventional methods, such as chromatography on a silica column and/or recrystallisation, compounds of the general formula (I) wherein $R_5$ does not represent a hydrogen atom,

- or directly with an acid chloride of the general formula (IX) to obtain, after isolation and purification by conventional methods, such as chromatography on a silica column and/or recrystallisation, compounds of the general formula (I) wherein $R_5$ is a hydrogen atom, which may then be N-alkylated according to conventional methods by a compound of the general formula (VII) to obtain, after isolation and where necessary purification by conventional methods, such as chromatography on a silica column and/or recrystallisation, compounds of the general formula (I) wherein $R_5$ does not represent a hydrogen atom,

- it being understood that when the compounds of the general formula (I) are not in the form of pure diastereoisomers they may be separated into their different diastereoisomers and/or be converted into salts with a pharmaceutically acceptable mineral or organic acid.

**2.** Process according to claim 1) for the preparation of the compound N- (4-phenyloctahydropyrido [2,1-c] [1,4]-oxazin-1-yl)-3,4,5-trimethoxybenzamide and also its isomers, isolated or in the form of a mixture, and their addition salts with a pharmaceutically acceptable mineral or organic acid.

**3.** Process according to claim 1) for the preparation of the compound (1R), (4R), (9aS)-N-(4-phenyloctahydropyrido [2,1-c] [1,4] oxazin-1-yl)-3,4,5-trimethoxybenzamide and also its addition salts with a pharmaceutically acceptable mineral or organic acid

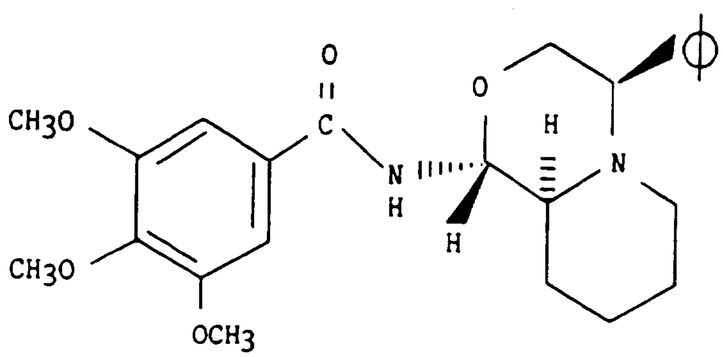

**4.** Process according to claim 1) for the preparation of the compound N-(4-phenyloctahydropyrido[2,1-c] [1,4]-oxazin-1-yl)-2,6-dimethoxy-3,5-dibromobenzamide and also its isomers, isolated or in the form of a mixture, and their addition salts with a pharmaceutically acceptable mineral or organic acid.

**5.** Process according to claim 1) for the preparation of the compound N-(1-methyl-4-phenyloctahydropyrido[2,1-c] [1,4]oxazin-1-yl)-3,4,5-trimethoxybenzamide and also its isomers, isolated or in the form of a mixture, and their addition salts with a pharmaceutically acceptable mineral or organic acid.

**6.** Process according to claim 1) for the preparation of the compound N-(4-methyl-3-phenyloctahydropyrido[2,1-c] [1,4]oxazin-1-yl)-3,4,5-trimethoxybenzamide and also its isomers, isolated or in the form of a mixture, and their addition salts with a pharmaceutically acceptable mineral or organic acid.

**7.** Process for the preparation of compounds of the general formula (VI) :

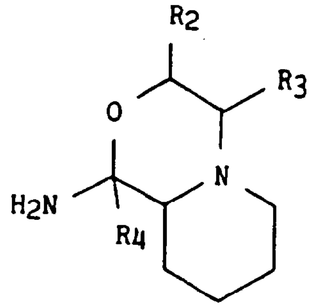

(VI)

wherein $R_2$, $R_3$ and $R_4$ are as defined for formula (I) in claim 1), as well as their isomers, isolated or in the form of a mixture, and their addition salts with a mineral or organic acid, which can be used as synthesis intermediates for the preparation of compounds of the general formula (I),
characterised in that
an amino alcohol of the general formula (II) :

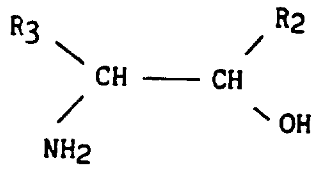

(II),

wherein $R_2$ and $R_3$ are as defined for compounds of the general formula (I), is reacted in aqueous medium, in the presence of a weak acid and at a temperature of from 0 to 20°C, with glutaraldehyde and then, with or without intermediate isolation, with potassium cyanide at a temperature of from 10°C to 30°C to obtain, after isolation and purification by conventional methods, such as chromatography on a silica column and/or recrystallisation, a bicyclic compound of the general formula (III) :

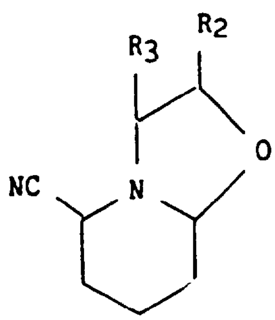

(III)

wherein $R_2$ and $R_3$ are as defined for compounds of the general formula (I), which may optionally be reacted, in an aprotic medium and at a temperature of from -78°C to room temperature, with an organolithium compound of the general formula (IV) :

$$R'_4Li \quad (IV)$$

wherein $R'_4$ has the same meaning as $R_4$ in the compounds of the general formula (I), with the proviso that $R'_4$ cannot represent a hydrogen atom, to obtain an imine of the general formula (V) :

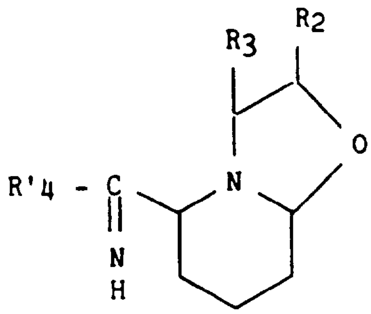

(V)

wherein $R_2$ and $R_3$ are as defined for compounds of the general formula (I) and $R'_4$ is as defined for compounds of the general formula (IV), which compound of formula (V) may be reacted, in the same manner as the nitriles of the general formula (III), with a metallic hydride in aprotic medium to obtain, after isolation and where necessary purification by conventional methods, such as chromatography on a silica column and/or recrystallisation, compounds of the general formula (VI) :

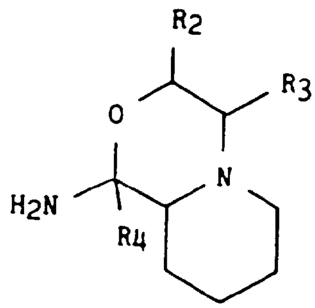

(VI)

wherein $R_2$, $R_3$, $R_4$ are as defined for compounds of the general formula (I) (the configuration of the asymmetric carbon atoms of the compounds of the general formulae (III) and (V) being carried over to the asymmetric carbon atoms of the compounds of the general formula (VI)).

**8.** Process for the preparation of a pharmaceutical composition comprising as active ingredient a compound prepared in accordance with any one of claims 1) to 6), alone or in combination with one or more pharmaceutically acceptable inert non-toxic excipients or carriers.

**9.** Process for the preparation of a pharmaceutical compositions according to claim 8) presented in a form suitable especially for the treatment of psycho-behavioural disorders.

**Claims for the following Contracting State : GR**

**1.** Process for the preparation of 1-acylaminooctahydropyrido[2,1-c] [1,4]oxazines of the general formula (I):

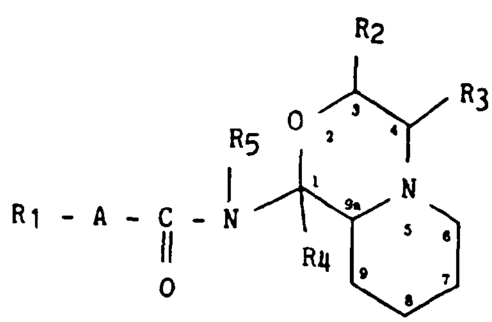

(I)

wherein :

- $R_1$ represents a hydrogen atom or a mono- or bicyclic system selected from phenyl, naphthyl, pyridyl, thienyl, furyl, quinolyl, isoquinolyl, indolyl, indolinyl, perhydroindolyl and benzofuryl, that is optionally substituted,

- A represents a σ bond or a straight-chained or branched, saturated or unsaturated, alkyl group having from 1 to 4 carbon atoms, with the proviso that when $R_1$ represents a hydrogen atom A cannot represent a σ bond,

- $R_2$ and $R_3$ each represents, independently of the other,

  - a hydrogen atom,
  - an optionally substituted, straight-chained or branched alkyl group having from 1 to 6 carbon atoms,
  - a $-(CH_2)_nB$ group in which n may be 0, 1, 2 or 3 and B represents a substituted or unsubstituted, saturated, unsaturated or aromatic monocyclic system having from 5 to 7 ring members,

  or $R_2$ and $R_3$ form together with the carbon atoms to which they are bonded an optionally substituted saturated cyclic system having from 5 to 7 ring members,

- $R_4$ and $R_5$ each represents, independently of the other, a hydrogen atom, a straight-chain or branched alkyl group having from 1 to 4 carbon atoms or a $-(CH_2)_nB$ group as defined in the description of $R_2$ and $R_3$,

the term "substituted" associated with "mono- or bicyclic" systems denotes that the rings may be substituted by one or more (4 maximum) identical or different groups selected from branched and unbranched lower alkyl having from 1 to 4 carbons atoms, nitro, amino, branched and unbranched lower alkoxy having from 1 to 4 carbon atoms, hydroxy, trifluoromethyl, halogen and $SO_2R_6$,
the term "substituted" associated with the term "alkyl" denotes that that group may be substituted by one or more straight-chained or branched lower alkoxy groups having from 1 to 4 carbon atoms,

- $R_6$ represents a branched or unbranched lower alkyl group having from 1 to 4 carbon atoms, a phenyl group (optionally substituted by one or more groups straight-chained or branched lower alkyl or alkoxy having from 1 to 4 carbon atoms, trifluoromethyl or halogen) or a $-NR_7R_8$ group in which $R_7$ and $R_8$ may each represent, independently of the other, a hydrogen atom or a branched or unbranched lower alkyl group having from 1 to 4 carbon atoms,

- their isomers, diastereoisomers and enantiomers, isolated or in the form of a mixture,

- and their addition salts with a pharmaceutically acceptable mineral or organic acid,

characterised in that :
an amino alcohol of the general formula (II) :

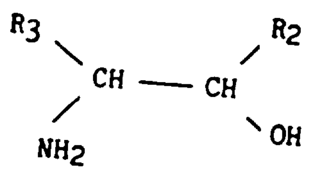

(II),

wherein $R_2$ and $R_3$ are as defined for compounds of the general formula (I), is reacted in aqueous medium, in the presence of a weak acid and at a temperature of from 0 to 20°C, with glutaraldehyde and then, with or without intermediate isolation, with potassium cyanide at a temperature of from 10°C to 30°C to obtain, after isolation and purification by conventional methods, such as chromatography on a silica column and/or recrystallisation, a bicyclic compound of the general formula (III) :

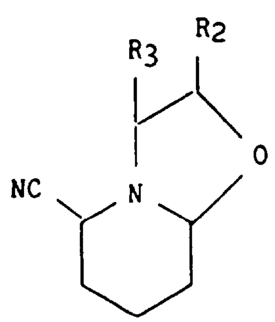

(III)

wherein $R_2$ and $R_3$ are as defined for compounds of the general formula (I), which may optionally be reacted, in an aprotic medium and at a temperature of from -78°C to room temperature, with an organolithium compound of the general formula (IV) :

$$R'_4Li \quad (IV)$$

wherein $R'_4$ has the same meaning as $R_4$ in the compounds of the general formula (I), with the proviso that $R'_4$ cannot represent a hydrogen atom, to obtain an imine of the general formula (V) :

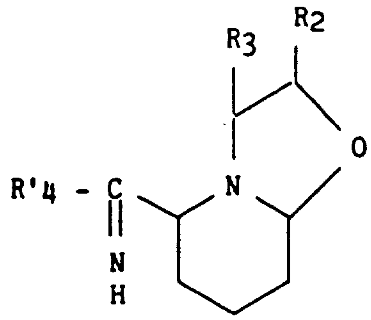

(V)

wherein $R_2$ and $R_3$ are as defined for compounds of the general formula (I) and $R'_4$ is as defined for compounds of the general formula (IV), which compound of formula (V) may be reacted, in the same manner as the nitriles of the general formula (III), with a metallic hydride in aprotic medium to obtain, after isolation and where necessary purification by conventional methods, such as chromatography on a silica column and/or recrystallisation, a compound of the general formula (VI) :

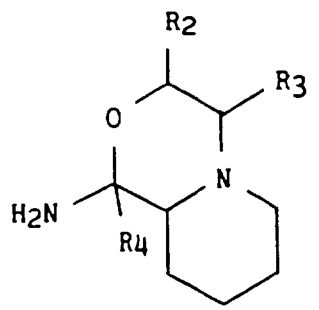

(VI)

wherein $R_2$, $R_3$, $R_4$ are as defined for compounds of the general formula (I) (the configuration of the asymmetric carbon atoms of the compounds of the general formulae (III) and (V) being carried over to the asymmetric carbon atoms of the compounds of the general formula (VI)), which may be reacted :

- either with a halogenated compound of the general formula (VII) :

  $$R'_5\text{-}X \quad (VII)$$

  wherein $R'_5$ has the same meaning as $R_5$ in the compounds of the general formula (I), with the proviso that $R'_5$ cannot represent a hydrogen atom, and X represents a halogen atom, to obtain, after isolation and where necessary purification by conventional methods, such as chromatography on a silica column and/or recrystallisation, a compound of the general formula (VIII) :

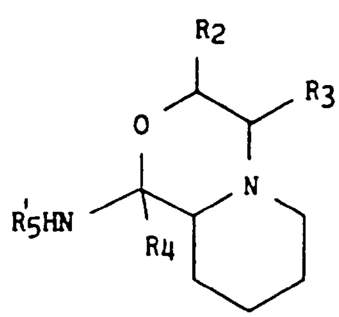

(VIII)

wherein $R_2$, $R_3$ and $R_4$ are as defined for compounds of the general formula (I) and $R'_5$ is as defined for the compounds of the general formula (VII), which is then reacted, optionally in the presence of a base, with an acid chloride of the general formula (IX) :

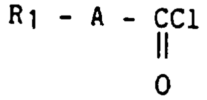

(IX)

wherein $R_1$ and A are as defined for compounds of the general formula (I) to obtain, after isolation and purification by conventional methods, such as chromatography on a silica column and/or recrystallisation, compounds of the general formula (I) wherein $R_5$ does not represent a hydrogen atom,

- or directly with an acid chloride of the general formula (IX) to obtain, after isolation and purification by conventional methods, such as chromatography on a silica column and/or recrystallisation, compounds of the general formula (I) wherein $R_5$ is a hydrogen atom, which may then be N-alkylated according to conventional methods by a compound of the general formula (VII) to obtain, after isolation and where necessary purification by conventional methods, such as chromatography on a silica column and/or recrystallisation, compounds of the general formula (I) wherein $R_5$ does not represent a hydrogen atom,

- it being understood that when the compounds of the general formula (I) are not in the form of pure diastereoisomers they may be separated into their different diastereoisomers and/or be converted into salts with a pharmaceutically acceptable mineral or organic acid.

**2.** Process according to claim 1) for the preparation of the compound N (4-phenyloctahydropyrido [2,1-c] [1,4]-oxazin-1-yl)-3,4,5-trimethoxybenzamide and also its isomers, isolated or in the form of a mixture, and their addition salts with a pharmaceutically acceptable mineral or organic acid.

**3.** Process according to claim 1) for the preparation of the compound (1R), (4R),(9aS)-N-(4-phenyloctahydropyrido [2,1-c] [1,4] oxazin-1-yl) -3,4,5-trimethoxybenzamide and also its addition salts with a pharmaceutically acceptable mineral or organic acid

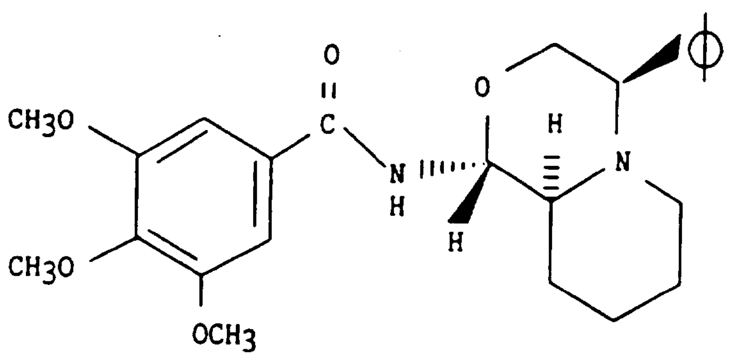

**4.** Process according to claim 1) for the preparation of the compound N-(4-phenyloctahydropyrido[2,1-c] [1,4]-oxazin-1-yl)-2,6-dimethoxy-3,5-dibromobenzamide and also its isomers, isolated or in the form of a mixture, and their addition salts with a pharmaceutically acceptable mineral or organic acid.

**5.** Process according to claim 1) for the preparation of the compound N-(1-methyl-4-phenyloctahydropyrido[2,1-c] [1,4]oxazin-1-yl)-3,4,5-trimethoxybenzamide and also its isomers, isolated or in the form of a mixture, and their addition salts with a pharmaceutically acceptable mineral or organic acid.

**6.** Process according to claim 1) for the preparation of the compound N-(4-methyl-3-phenyloctahydropyrido[2,1-cl [1,4]oxazin-1-yl)-3,4,5-trimethoxybenzamide and also its isomers, isolated or in the form of a mixture, and their addition salts with a pharmaceutically acceptable mineral or organic acid.

**7.** Process for the preparation of compounds of the general formula (VI) :

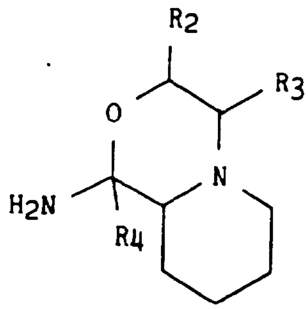

(VI)

wherein $R_2$, $R_3$ and $R_4$ are as defined for formula (I) in claim 1), as well as their isomers, isolated or in the form of a mixture, and their addition salts with a mineral or organic acid, which can be used as synthesis intermediates for the preparation of compounds of the general formula (I),
characterised in that
an amino alcohol of the general formula (II) :

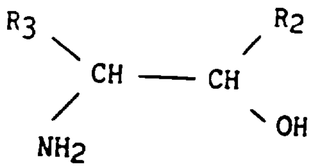

(II),

wherein $R_2$ and $R_3$ are as defined for compounds of the general formula (I), is reacted in aqueous medium, in the presence of a weak acid and at a temperature of from 0 to 20°C, with glutaraldehyde and then, with or without intermediate isolation, with potassium cyanide at a temperature of from 10°C to 30°C to obtain, after isolation and purification by conventional methods, such as chromatography on a silica column and/or recrystallisation, a bicyclic compound of the general formula (III) :

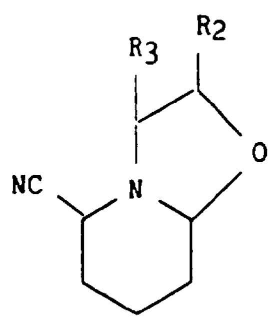

(III)

wherein $R_2$ and $R_3$ are as defined for compounds of the general formula (I), which may optionally be reacted, in an aprotic medium and at a temperature of from -78°C to room temperature, with an organolithium compound of the general formula (IV) :

$$R'_4Li \qquad (IV)$$

wherein $R'_4$ has the same meaning as $R_4$ in the compounds of the general formula (I), with the proviso that $R'_4$ cannot represent a hydrogen atom, to obtain an imine of the general formula (V) :

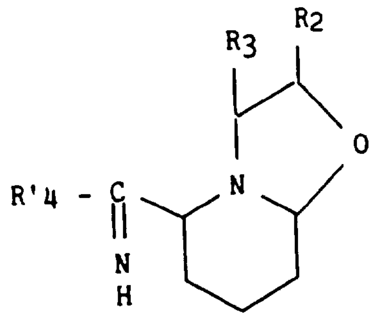

(V)

wherein $R_2$ and $R_3$ are as defined for compounds of the general formula (I) and $R'_4$ is as defined for compounds of the general formula (IV), which compound of formula (V) may be reacted, in the same manner as the nitriles of the general formula (III), with a metallic hydride in aprotic medium to obtain, after isolation and where necessary purification by conventional methods, such as chromatrography on a silica column and/or recrystallisation, compounds of the general formula (VI) :

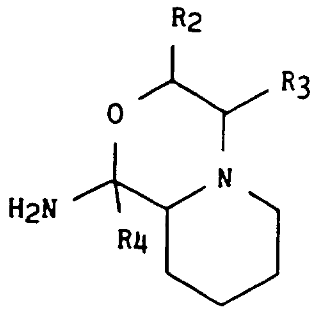

(VI)

wherein $R_2$, $R_3$, $R_4$ are as defined for compounds of the general formula (I) (the configuration of the asymmetric carbon atoms of the compounds of the general formulae (III) and (V) being carried over to the asymmetric carbon atoms of the compounds of the general formula (VI)).

**8.** Process for the preparation of a pharmaceutical composition comprising as active ingredient a compound prepared in accordance with any one of claims 1) to 6), alone or in combination with one or more pharmaceutically acceptable inert non-toxic excipients or carriers.

**9.** Process for the preparation of a pharmaceutical compositions according to claim 8) presented in a form suitable especially for the treatment of psycho-behavioural disorders.